# EUROPEAN PATENT APPLICATION

(11) **EP 4 545 702 A1**
(43) Date of publication of application: **30.04.2025**
(21) Application number: 23831793.7
(22) Date of filing: 20.06.2023
(51) Int. Cl.: D06F 58/26, D06F 58/10, D06F 58/32, D06F 73/02, D06F 39/00, A61L 2/07

(54) **CLOTHING TREATMENT APPARATUS**

(30) Priority: 30.06.2022 KR 20220080291
(71) Applicant: LG Electronics Inc., Yeongdeungpo-gu Seoul 07336 (KR)
(72) Inventor: KANG, Hyungha, Seoul 08592 (KR); KIM, Jaehyung, Seoul 08592 (KR)
(74) Representative: Vossius & Partner Patentanwälte Rechtsanwälte mbB
(86) International application number: PCT/KR2023/008517
(87) International publication number: WO 2024/005433

(57) **Abstract**

The present disclosure relates to a clothing treatment apparatus characterized in that, in order to enable water accommodated in a water tank to be supplied to a steam nozzle and the water supplied to the steam nozzle to be delivered to a steam generator, a steam supply part comprises: a water tank which is detachably provided in a machine room and stores water; a steam generator which accommodates a heating part that heats the water so as to generate steam; and a steam nozzle which is provided so as to communicate with the steam generator, and supplies the steam generated by the steam generator into an inner case, wherein the steam nozzle receives the water accommodated in the water tank and delivers same to the steam generator.

## Description

### [Technical Field]

The present disclosure relates to a laundry treating apparatus. More specifically, the present disclosure relates to a laundry treating apparatus that may remove wrinkles from laundry and sterilize the laundry by supplying steam into the laundry treating apparatus.

### [Background]

In general, a laundry treating apparatus is a concept that includes a washing machine that soaks laundry in water so as to be in a wet state and then removes foreign substances via a chemical action of detergent and a physical action such as drum rotation, and a dryer that dries the wet laundry using hot air and steam.

However, recently, a laundry manager that keeps the laundry in a comfortable and clean state without soaking the laundry in a dry state in water has appeared. Such laundry manager is able to perform a refreshing cycle by supplying steam or hot air while the laundry is mounted to deodorize the laundry and dry or sterilize the laundry.

Such laundry manager is able to optionally add fragrance to the laundry, and has recently taken up a significant proportion in the laundry treating apparatus along with the washing machine and the dryer.

Because the laundry manager that performs the refreshing cycle of the laundry accommodates the dry laundry therein, a steam supply that supplies steam to the laundry is essential.

Examples of such existing laundry treating apparatus include Korean Patent Application Publication Nos. 10-2020-0057545 and 10-2011-0067832.

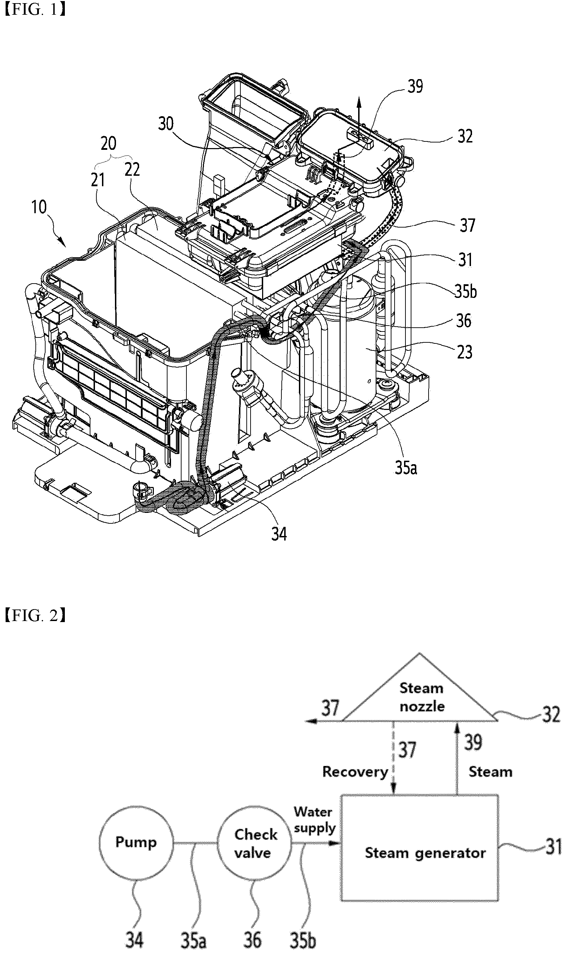

FIG. 1 shows a machine room structure that may be applied to an existing laundry treating apparatus.

The machine room of the existing laundry treating apparatus is disposed downward of an inner casing in which the laundry is mounted, and supplies hot air and steam to inside of the inner casing.

The machine room includes a circulation duct 10 that circulates air inside the inner casing, a heat exchanger assembly 20 that condenses and heats air circulated in the circulation duct 10, and a steam supply 30 that supplies steam into the inner casing.

The heat exchanger assembly 20 may include heat exchangers 21 and 22 that are accommodated in the circulation duct 10 and condense and heat air introduced from the inner casing, and a compressor 12 that supplies a refrigerant to the heat exchanger 11.

The steam supply 30 may include a steam generator 31 that generates steam as a heater to boil water is installed therein, and a steam nozzle 32 that receives steam generated from the steam generator 31 and supplies steam into the inner casing.

The steam supply 30 may include a steam pipe 39 that may guide steam generated from the steam generator 31 to the steam nozzle 32.

The steam nozzle 32 may be disposed upward of the steam generator 31 to utilize a force of steam rising because of a density difference, and the steam pipe 39 may allow the steam generator 31 and the steam nozzle 32 to be in direct communication with each other.

The steam supply 30 may include a separate water supply tank that may supply water to the steam generator 31, and may further include a supply pipe 33 that supplies water from the water supply tank, and a water pump 34 that may supply water from the supply pipe 33 to the steam supply 30.

Generally, the existing laundry treating apparatus may supply water from the water supply tank to the steam generator 31 to generate steam in the steam supply 30. Accordingly, the steam generator 31 may directly receive water from the water pump 34, and quickly secure a water level for heating steam in the steam generator 31.

To this end, the steam supply 30 of the existing laundry treating apparatus further includes a water supply pipe 35 that may directly supply water supplied from the water pump 34 to the steam generator 31. The water supply pipe 35 allows the water pump 34 and the steam generator 31 to be in direct communication with each other.

In one example, the existing laundry treating apparatus has the steam generator 31 positioned upward of the water pump 34, so that there is a high possibility that water remaining in the steam generator 31 or water contained inside the water supply pipe 35 will flow back to the water pump 34.

Therefore, the existing laundry treating apparatus needs to additionally place a check valve 36 on the water supply pipe 35. As a result, a flow channel structure for supplying water becomes complicated, and there is inconvenience of having to prepare or manage a separate component such as the check valve 36.

For example, there is a limitation that the water supply pipe 35 has to be composed of a first water supply pipe 35a that connects the water pump 34 with the check valve 36, and a second water supply pipe 35a that connects the check valve 36 with the steam generator 31 on the check valve 36.

In one example, steam may be completely discharged from the steam nozzle 32, but some of steam may condense and turn into water. In this regard, when the condensed water flows back via the steam pipe 39, it hinders a movement of steam supplied from the steam generator 31.

To prevent this, the existing laundry treating apparatus may further include a discharge pipe 37 that may separately discharge condensate generated from the steam nozzle 32.

The discharge pipe 37 may be coupled to a lower end of the steam nozzle 32 and may be spaced apart from the steam pipe 39 such that water condensed in the steam nozzle 32 may be induced to be discharged by its own weight without hindering the movement of steam. The discharge pipe 37 may also be connected to the steam generator 31 so as to recycle condensed water.

However, condensate generated from the steam nozzle 32 is not generated in a large amount to cause noticeable water buildup within the steam nozzle 32. As a result, the existing laundry treating apparatus is not able to automatically clean the steam nozzle 32 when the foreign substances are generated inside the steam nozzle 32 or the steam nozzle 32 is contaminated by bacteria or the like.

Moreover, most existing laundry treating apparatuses are designed to discard condensate discharged via the discharge pipe 37 by directly delivering the same to a place where condensed water is collected in the heat exchangers 11 and 12, instead of supplying the same to the steam generator 31.

As a result, water condensed in the steam nozzle 32 may be prevented from separately flowing into the steam generator 31 in addition to the water supply pipe 35, thereby preventing the water level of the steam generator 31 from varying unintentionally or the temperature inside the steam generator 31 from varying.

In addition, when the inside of the steam nozzle 32 is contaminated by bacteria, mold, or the foreign substances, water inside the steam generator 31 may be prevented from rotting because of the contaminated water.

However, there is an inefficiency in that water condensed in the steam nozzle 32 has to be discarded even though it has never been discharged as steam.

FIG. 2 is a schematic diagram of a flow channel structure of such existing laundry treating apparatus.

Because the existing laundry treating apparatus should be equipped to directly supply water from the water pump 34 to the steam generator 31, there is a limitation that the water supply pipe 35 connecting the water pump 34 with the steam generator 31 should be disposed and the check valve 36 should be installed to prevent water from the steam generator 31 from flowing back to the water pump 34.

Because of the check valve 36, the existing laundry treating apparatus has a problem in that the water supply pipe 35 also has to be divided into the plurality of parts, such as the first water supply pipe 35a and the second water supply pipe 35a, based on the check valve 36.

In particular, the existing laundry treating apparatus is vulnerable to internal contamination because only steam is supplied to the steam nozzle 32 and separate water is not able to be supplied thereto.

In addition, the existing laundry treating apparatus has a problem in that all of the water supply pipe that supplies water to the steam generator 31, the steam pipe that discharges steam, and a recovery pipe that recovers condensate have to be installed, which makes the flow channel structure very complicated and reduces accuracy of flow channel control.

In addition, in the existing laundry treating apparatus, when water condensed in the steam nozzle 32 is not recovered to the steam generator 31 but is discarded, water is wasted and a water level of the water supply tank rapidly decreases.

### [Summary]

### [Technical Problem]

The present disclosure is to provide a laundry treating apparatus that may simplify a flow channel or hose structure related to a steam supply that supplies steam to an inner casing where laundry is mounted.

The present disclosure is to provide a laundry treating apparatus that may omit a check valve in the steam supply.

The present disclosure is to provide a laundry treating apparatus that may wash a steam nozzle that supplies steam to an inner casing with water supplied from a water pump.

The present disclosure is to provide a laundry treating apparatus that may recycle steam condensed in the steam nozzle.

### [Technical Solutions]

To solve the above-described problems, the present disclosure provides a laundry treating apparatus that supplies water accommodated in a water supply tank to a steam nozzle and transfers water supplied to the steam nozzle to a steam generator.

The steam nozzle may be disposed upward of the steam generator.

The steam nozzle may directly supply, to the steam generator, water supplied to the steam nozzle via a recovery hose.

The recovery hose may have both ends disposed higher than other portions to prevent water supplied to the steam generator from flowing back to the steam nozzle.

The steam supply may include a water pump that supplies water stored in the water supply tank to the steam nozzle, and a water supply pipe connected to the water pump may be directly connected to the steam nozzle.

The steam supply may further include a steam hose transferring steam generated from the steam generator to the steam nozzle. The steam hose may be separately disposed by being separated and spaced apart from the water supply pipe and the recovery hose.

The steam nozzle may include a supply container providing an internal space for receiving water or steam, a first pipe extending from the supply container to receive water, and a second pipe extending from the supply container to deliver water to the steam generator, and the second pipe may be positioned downward of the first pipe.

The steam nozzle may further include a third pipe extending from the supply container to receive steam, and the third pipe may be disposed downward of the first pipe.

The supply container may include a container side surface having the first pipe disposed thereon and defining the internal space, and a bottom surface extending from a lower portion of the container side surface and having the second pipe and the third pipe disposed thereon, and the second pipe may be disposed closer to the first pipe than to the third pipe.

The supply container may include a container side surface having the first pipe disposed thereon and defining the internal space, and a bottom surface extending from a lower portion of the container side surface and having the second pipe and the third pipe disposed thereon. The bottom surface may be disposed in an inclined or stepped manner such that the second pipe is disposed lower than the third pipe.

The supply container may include a separation partition wall protruding from the bottom surface to partition the second pipe and the third pipe from each other.

The separation partition wall may extend from the bottom surface to surround the third pipe.

The steam nozzle may further include a container cover coupled to an upper portion of the supply container and transmitting steam into the inner casing.

The container cover may include a steam spray hole discharging steam to the outside of the supply container, and a separating plate extending from one side of the steam spray hole and spaced apart from an inner surface of the supply container to obstruct movement of steam.

The separating plate may be disposed between the second pipe and the third pipe.

The separating plate may be disposed between the steam spray hole and the third pipe.

The separating plate may be disposed closer to the third pipe than to the second pipe.

### [Advantageous Effects]

The present disclosure may simplify the flow channel or hose structure related to the steam supply that supplies steam to the inner casing where the laundry is mounted.

The present disclosure may omit the check valve in the steam supply.

The present disclosure may wash the steam nozzle that supplies steam to the inner casing with water supplied from the water pump.

The present disclosure may recycle steam condensed in the steam nozzle.

### [Brief Description of the Drawings]

FIG. 1 shows an existing laundry treating apparatus.
FIG. 2 schematically shows a steam supply flow channel of an existing laundry treating apparatus.
FIG. 3 shows an outer appearance of a laundry treating apparatus of the present disclosure.
FIG. 4 shows a machine room structure of a laundry treating apparatus of the present disclosure.
FIG. 5 shows a machine room base structure of a laundry treating apparatus of the present disclosure.
FIG. 6 shows a circulation duct structure of a laundry treating apparatus of the present disclosure.
FIG. 7 illustrates a shape of a circulation duct of a laundry treating apparatus of the present disclosure.
FIG. 8 is a cross-sectional view of the circulation duct.
FIG. 9 shows a structure of a reservoir of a laundry treating apparatus of the present disclosure in detail.
FIG. 10 is a cross-sectional view (S-S') of a circulation duct cut in a height direction.
FIG. 11 shows an inclined structure related to a reservoir.
FIG. 12 shows structures of the reservoir and a residual water treater.
FIG. 13 shows an embodiment of a residual water treater of a laundry treating apparatus of the present disclosure.
FIG. 14 shows an embodiment of a water cover.
FIG. 15 shows a state in which the water cover is installed in the circulation duct.
FIG. 16 shows a detailed structure of a water cover.
FIG. 17 shows a controller installation portion structure disposed on a base of a laundry treating apparatus of the present disclosure.
FIG. 18 shows a structure of an air discharger 323 of a laundry treating apparatus of the present disclosure.
FIG. 19 shows a structure of a base cover of a laundry treating apparatus of the present disclosure.
FIG. 20 shows a structure of an outside air duct.
FIG. 21 shows a flow of air flowing through the circulation duct.
FIG. 22 shows an installation structure of a steam supply.
FIG. 23 shows a detailed structure of the steam supply.
FIG. 24 shows inside of the steam generator.
FIG. 25 shows a steam supply in which the heater assembly is installed.
FIG. 26 shows a water supply flow channel structure of a steam supply of the present disclosure.
FIG. 27 is a schematic diagram of a water supply flow channel of the present disclosure.
FIG. 28 shows a water supply structure of a steam nozzle and a steam supply of the present disclosure.
FIG. 29 shows a detailed structure of a steam nozzle of the present disclosure.
FIG. 30 shows another embodiment of a steam nozzle of the present disclosure.

### [Best Mode]

Hereinafter, embodiments disclosed herein will be described in detail with reference to the attached drawings. In the present document, identical or similar components are assigned identical or similar reference numerals even in different embodiments, and descriptions thereof are replaced with the first description. A singular expression used herein includes a plural expression unless the context clearly indicates otherwise. In addition, when describing the embodiments disclosed herein, when it is determined that a detailed description of a related known technology may obscure the gist of the embodiments disclosed herein, the detailed description thereof will be omitted. In addition, it should be noted that the attached drawings are only intended to facilitate easy understanding of the embodiments disclosed herein, and the technical ideas disclosed herein should not be construed as being limited by the attached drawings.

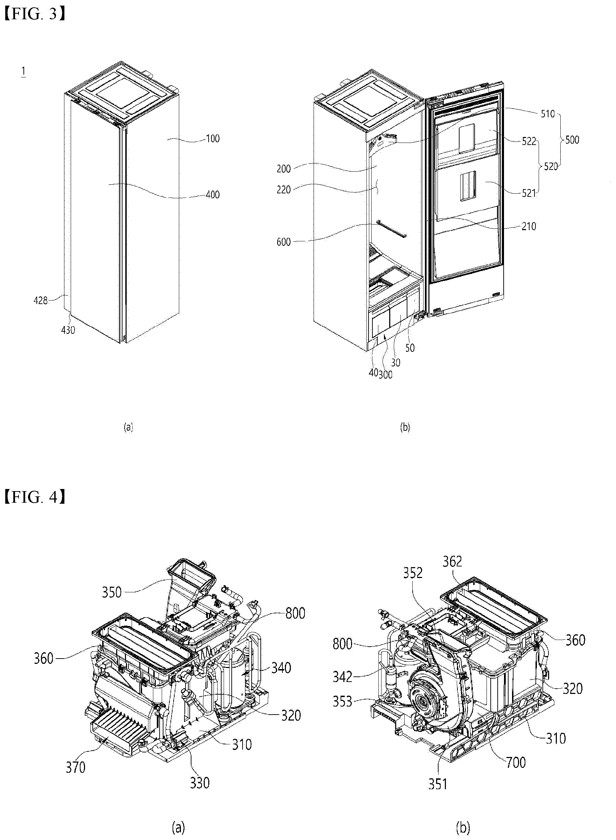

FIG. 3 shows an outer appearance of a laundry treating apparatus 1 of the present disclosure.

Referring to (a) in FIG. 3, the laundry treating apparatus of the present disclosure may include a cabinet 100 forming the outer appearance thereof, and a door 400 pivotably coupled to the cabinet 100.

The door 400 may include a main body 410 forming a front surface of the cabinet 100, and an installation body 420 extending from one side of the main body 410 and on which a display for displaying information of the laundry treating apparatus may be installed.

The installation body 420 may form a step 430 from the main body 410 in a rearward direction of the cabinet 100.

In one example, at least a portion of the installation body 420 may be disposed at the rear of the main body 410 so as to overlap the main body 410 in a front and rear direction. Accordingly, the step 430 may serve as a handle.

The installation body 420 may be made of a material or may have a color different from that of the main body 410. In addition, the installation body 420 may be made of a translucent material through which light emitted from the display may be transmitted.

Referring to (b) in FIG. 3, an inner casing 200 having an accommodating space 220 for accommodating laundry may be disposed inside the cabinet 100. The inner casing 200 may have an opening 210 at a front side thereof through which the laundry enters and exits, and the opening 210 may be shielded by the door 400.

The inner casing 200 may be made of a plastic resin-based material, and may be made of a reinforced plastic resin-based material that does not deform even with air at a temperature higher than a room temperature or heated air (hereinafter, hot air) and steam or moisture.

The inner casing 200 may have a height greater than a width. Accordingly, the laundry may be accommodated in the accommodating space 220 without being folded or wrinkled.

The laundry treating apparatus 1 of the present disclosure may include a mounter 500 that may mount the laundry in the accommodating space 220 of the inner casing 200.

The mounter 500 may include a hanger 510 disposed on a top surface of the inner casing 200 to mount the laundry thereon.

When the laundry is mounted on the hanger 510, the laundry may be placed in a state of floating in air inside the accommodating space 220.

In one example, the above mounter 500 may further include a pressurizer 520 that may be coupled to an inner surface of the door 400 to fix the laundry.

The hanger 510 may be formed in a bar shape disposed along a width direction of the inner casing 200 to support a clothes hanger on which the laundry is mounted. In addition, as illustrated, the hanger 510 may be formed in the clothes hanger shape to enable the laundry to be directly mounted thereon.

The laundry treating apparatus of the present disclosure may further include an oscillator that vibrates the hanger 510 to remove foreign substances such as fine dust attached to the laundry.

The mounter 500 may include the pressurizer 520 that may be disposed on the door 400 and may pressurize and fix the laundry. The pressurizer 520 may include a support 522 that is fixed to the inner surface of the door 400 and supports one surface of the laundry, and a press 521 that pressurizes the laundry supported on the support 522.

The press 521 may move toward or away from the support 522. For example, the press 521 may be pivotably disposed on the support 522 or the inner surface of the door 400.

Therefore, the press 521 and the support 522 may pressurize both surfaces of the laundry to remove wrinkles from the laundry and create intended creases.

The laundry treating apparatus of the present disclosure may be equipped with a machine room 300 in which various apparatuses that may supply at least one of hot air and steam to the accommodating space 220 or purify or dehumidify outside air of the cabinet 100 are installed.

The machine room 300 may be disposed separately or partitioned from the inner casing 200, but may be in communication with the inner casing 200.

The machine room 300 may be disposed under the inner casing 200. Accordingly, when hot air and steam having low specific gravities are supplied to the inner casing 200, hot air and steam may be naturally supplied to the laundry.

The machine room 300 may include a heat supply 340 that may supply hot air into the inner casing 200. The heat supply 340 may be equipped as a heat pump system, or may be equipped as a heater that directly heats air with electric energy.

When the heat supply 340 is equipped as the heat pump system, it may dehumidify and heat air discharged from the inner casing 200 again and supply air to the inner casing 200. A detailed structure thereof will be described later.

The machine room 300 may include a steam supply 800 that may supply steam into the inner casing 200. The steam supply 800 may be equipped to directly supply steam into the inner casing 200. A detailed structure thereof will be described later.

To this end, the inner casing 200 may include a plurality of through-holes 230 that extend through one surface thereof and are in communication with the machine room 300.

Via the through-holes 230, air in the accommodating space 220 may be supplied to the machine room 300, and at least one of hot air and steam generated in the machine room 300 may be supplied to the accommodating space 200.

The through-holes 230 may include an inflow hole 231 extending through a bottom surface of the inner casing 200 and through which air inside the inner casing 200 is discharged or sucked into the machine room 300, and an exhaust hole 232 extending through the bottom surface of the inner casing 200 and through which hot air generated in the machine room 300 is discharged.

The exhaust hole 232 may be defined in the bottom surface of the inner casing 200 so as to be biased toward a rear surface of the inner casing 200. For example, the exhaust hole 232 may be defined so as to be inclined with respect to the ground between the bottom surface and the rear surface of the inner casing 200 and to face the hanger 510.

In addition, the inflow hole 231 may be defined in the bottom surface of the inner casing 200 so as to be biased forward. Accordingly, the inflow hole 231 may be defined to be spaced apart from the exhaust hole 232.

The through-holes 230 may include a steam hole 233 to which steam generated from the steam supply 800 is supplied. The steam hole 233 may be defined on one side of the exhaust hole 232.

In one example, a water supply tank 30 that may supply water to the steam supply 800 and a drainage tank 40 in which condensate condensed in the heat supply 340 is collected may be disposed at a front side of the machine room 300.

The water supply tank 30 and the drainage tank 40 may be detachably disposed at the front side of the machine room 300. Accordingly, the laundry treating apparatus 1 of the present disclosure may be freely installed without being restricted by a water supply source or a drainage source.

In one example, a drawer 50 that is extended forward and has a separate accommodating space may be further disposed at the front side of the machine room 300. The drawer 50 may store a steam generator or an iron therein.

FIG. 4 shows a machine room structure of a laundry treating apparatus of the present disclosure.

(a) in FIG. 4 is a front view of the machine room 300, and (b) in FIG. 4 is a rear view of the machine room 300.

Inside the machine room 300, components for supplying hot air to a laundry treatment space, circulating air inside the laundry treatment space, supplying steam to the laundry treatment space, or purifying air outside the cabinet may be disposed.

The machine room 300 may include a base 310 that defines a space in which various apparatuses are supported or installed. The base 310 may provide an area size for the various apparatuses to be installed.

The base 310 may be installed with a circulation duct 320 through which air introduced from the inner casing 200 or the outside of the cabinet 100 flows.

The circulation duct 320 may be formed in a casing shape with an open top surface, and some components of the heat supply 340 may be installed inside the circulation duct 320.

When the heat supply 340 is equipped as the heat pump system, the circulation duct 320 may include heat exchangers 341 and 342 to be described below and a compressor 342 that supplies a high-temperature and high-pressure refrigerant to the heat exchanger.

The heat exchangers 341 and 342 may be accommodated inside the circulation duct 320 to cool and dehumidify air flowing through the circulation duct 320, or may heat the air to generate hot air.

When the circulation duct 320 is equipped to suck air outside the cabinet 100, an outside air duct 370 that sucks outside air may be installed in front of the circulation duct 320.

The circulation duct 320 may be in communication with the outside air duct 370 and may selectively suck outside air.

The water supply tank and the drainage tank may be detachably coupled to a front surface of the circulation duct 320. The water supply tank 30 and the drainage tank 40 may be seated and disposed on the outside air duct 370.

The circulation duct 320 may be coupled to the base 310, but may be formed integrally with the base 310. For example, the base 310 and the circulation duct 320 may be manufactured via injection-molding.

The machine room 300 may include a base cover 360 that allows the circulation duct 320 and the inflow hole 231 to be in communication with each other.

The base cover 360 may be coupled to an upper portion of the circulation duct 320 to guide air sucked from the inflow hole 231 into the circulation duct 320.

The base cover 360 may block air inside the circulation duct 320 from being discharged to the outside by shielding a top surface of the circulation duct 320. A lower portion of the base cover 360 and the top surface of the circulation duct 320 may form one surface of a flow channel of the circulation duct 320.

The base cover 360 may include an inlet 362 connecting the inflow hole 231 with the circulation duct 320. The inlet 362 may be formed in a duct shape to serve as an intake duct that delivers air inside the inner casing 200 to the circulation duct 320.

The steam supply 800 that is connected to the water supply tank 30 to receive water, generate steam, and supply steam to the inner casing 200 may be installed in the machine room 300. The steam supply 800 may be seated and disposed on the base cover 360.

The steam supply 800 may be disposed at the rear of the inlet 362.

The machine room 300 may include a fan installation portion 350 that is in communication with the circulation duct 320 and the inner casing 200. The fan installation portion 350 may include a blower fan 353 that provides power for air inside the circulation duct 320 to flow in one direction, and a fan housing 351 that accommodates the blower fan 353 therein and is coupled to or extended from the circulation duct 320.

The fan installation portion 350 may include an exhaust duct 352 that allows the circulation duct 320 and the exhaust hole 232 to be in communication with each other.

The exhaust duct 352 may extend from the fan housing 351 toward the exhaust hole 232 with an area size of a cross-section corresponding to the exhaust hole 232.

As a result, air inside the inner casing 200 may be introduced via the base cover 360, then pass through the circulation duct 320, and then be supplied back into the inner casing 200 via the fan installation portion 350.

In one example, the base 310 may include a compressor installation portion 313 in which the compressor 342 that supplies the refrigerant to the heat exchangers 341 and 343 is installed. The compressor installation portion 313 may be disposed outside the circulation duct 320.

In addition, a controller or a control panel 700 that controls the laundry treating apparatus of the present disclosure may be installed on the base 310.

The base 310 may include a controller installation portion 312 that defines a space in which the controller 700 may be inserted under the circulation duct 320.

The controller 700 may control all electronically controlled components such as the compressor 342, the steam supply 800, and the blower fan 353.

Because the controller 700 is inserted and supported in the base 310, vibration or impact applied to the controller 700 may be buffered. In addition, because the controller 700 is disposed close to all of the electronic components, occurrence of control errors such as noise may be minimized.

In addition, the steam supply is disposed on the circulation duct 320, and the controller 700 is disposed under the circulation duct 320. Therefore, the circulation duct 320 may be formed in a straight duct shape between the steam supply 800 and the controller 700. Therefore, a flow resistance of air passing through the circulation duct 320 may be minimized.

The circulation duct 320, the outside air duct 370, the steam supply 800, the controller 700, and the heat supply 340 may be equipped in a module format on the base 310.

Accordingly, the base 310 may be easily installed and maintained while being extended forward from and retracted rearward into the machine room 300.

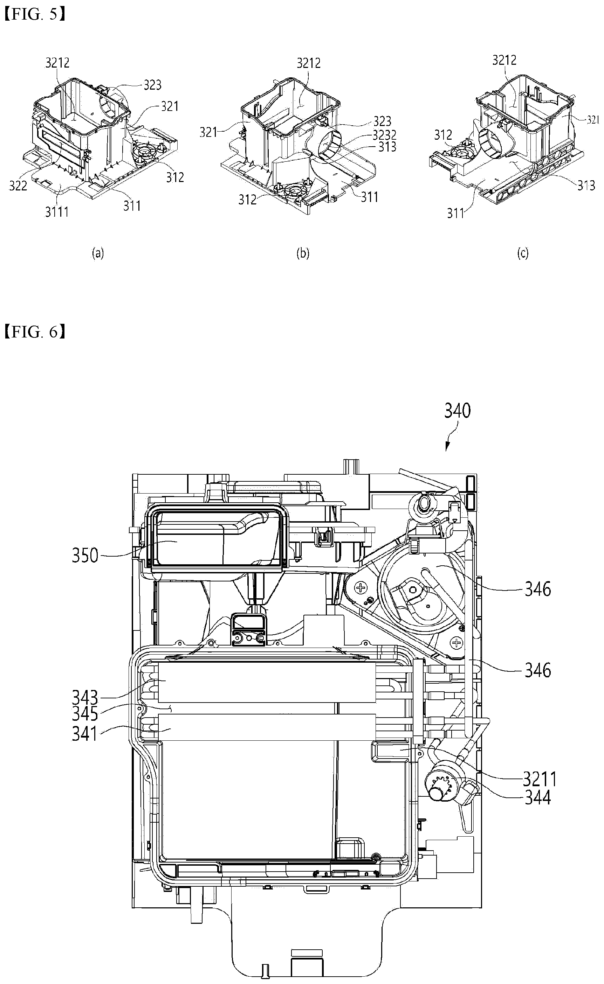

FIG. 5 shows a machine room base structure of a laundry treating apparatus of the present disclosure.

(a) in FIG. 5 is a front perspective view of the base 310, and (b) and (c) in FIG. 5 are rear perspective views of the base 310.

The base 310 may be installed on a base plate forming a bottom surface of the laundry treating apparatus. The base 310 may itself form the bottom surface of the laundry treating apparatus.

The base 310 may include a base bottom 311 forming a support surface. The base bottom 311 may form the bottom surface of the laundry treating apparatus. In addition, the base bottom 311 may be installed on a top surface of a bottom surface of the cabinet 100 forming the bottom surface of the laundry treating apparatus.

The base 310 may be integrally formed with the circulation duct 320 forming at least a portion of the flow channel through which air flows. The circulation duct 320 may be formed by extending upward from the base bottom 311.

The circulation duct 320 may include a duct body 321 that extends from the base bottom 311 to form the flow channel, a heat exchanger installation portion 3212 that provides a space in which an evaporator 341 or a condenser 343 is installed inside the duct body 321, and an air discharger 323 that is disposed at the rear of the duct body 321 and through which air of the duct body 321 is discharged.

The air discharger 323 may be formed in a pipe shape that extends rearward from the duct body 321. A diameter of the air discharger 323 may be smaller than a width of the duct body 321.

The air discharger 323 may be connected to a fan housing 350. Air discharged from the air discharger 323 may be guided into the inner casing 200 via the fan housing 350.

The circulation duct 320 may include an outside air intake portion 322 formed by extending through a front surface of the duct body 321.

The outside air intake portion 322 may be in communication with the outside air duct 370. The outside air duct 370 may be supported by being seated in front of the outside air intake portion 322.

The circulation duct 320 may include a damper that opens and closes the outside air intake portion 322. Introduction of outside air into the circulation duct 320 may be allowed or blocked via the opening and closing of the damper.

The base 310 may include a compressor installation portion 312 that provides a space in which the compressor 342 is installed. The compressor installation portion 312 may be formed at one side of the base bottom 311 and may be formed integrally with the base bottom 311.

The compressor installation portion 312 may be formed with a protrusion that may support the compressor 342. The compressor installation portion 312 may be disposed to be biased rearward on the base 310. The compressor installation portion 312 may be disposed to at least partially overlap the air discharger 323 in a width direction.

The compressor installation portion 312 may be installed with a buffer member that reduces vibration transmitted from the compressor 342. The buffer member may be fixed to the protrusion.

The base 310 may include a controller installation portion 313 in which the controller 700 is installed. The controller installation portion 313 may be formed between the base bottom 311 and the circulation duct 320. The controller installation portion 313 may be formed between the base bottom 311 and a bottom surface of the circulation duct 320. The controller installation portion 313 may be formed in a duct shape in which one of a front side and a rear side is open under the circulation duct 320.

A structure of the controller installation portion 313 will be described later.

FIG. 6 shows a circulation duct structure of a laundry treating apparatus of the present disclosure.

The circulation duct 320 may extend upward from the base bottom to form the flow channel through which air flows. The circulation duct 320 may include the heat exchanger installation portion 3212 that provides the space in which the evaporator 341 and the condenser 342 are installed. The heat exchanger installation portion 3212 may be disposed inside the duct body 321.

The duct body 321 may have an open top surface. The condenser 343 and the evaporator 341 may be inserted and installed via the opening of the duct body 321.

The opening of the duct body 321 may be shielded by the base cover 360, and the base cover 360 and the duct body 321 may form the flow channel of a circulation duct 320.

The front surface of the duct body 321 may be disposed to be spaced rearwardly apart from a front end of the base bottom 311.

As a result, the base bottom 311 may secure a support surface 3111 on which at least one of the water supply tank 30 or the drainage tank 40 and the outside air duct 370 described above is installed and supported.

In one example, the heat supply 340 may include the evaporator 341 that is installed inside the circulation duct 320 and is equipped as a heat exchanger for cooling and dehumidifying air introduced into the circulation duct 320, the condenser 343 that is equipped as a heat exchanger for heating air that has passed through the evaporator 341 to generate hot air, the compressor 342 that supplies the refrigerant for heat exchange with air to the condenser 343 and is disposed outside the circulation duct 320, and an expansion valve 344 that expands and cools the refrigerant that has passed through the condenser 343.

In one example, because the duct body 321 is integrally formed with the base 310, a height of the heat exchanger installation portion 3212 may be secured more, and heights of the condenser 343 and the evaporator 341 may also be increased.

As a result, widths in the front and rear direction of the condenser 343 and the evaporator 341 may be reduced, so that the number of refrigerant pipes passing through the condenser and the evaporator may be reduced. Accordingly, there is an effect of reducing a flow loss of air passing through the condenser and the evaporator.

In one example, a sum of a length of the evaporator 341 and a length of the condenser 343 may be smaller than a length of the heat exchanger installation portion 3212. Accordingly, the length in the front and rear direction of the heat exchanger installation portion 3212 may be equal to or smaller than half of a length of the duct body 321.

Therefore, because the heat exchanger installation portion 3212 may be sufficiently spaced apart from the outside air intake portion 322, sufficient space for outside air and air inside the inner casing 200 to be introduced into the circulation duct 320 may be secured.

In one example, the inside of the duct body 321 may include an installation partition wall 3211 that separates the heat exchanger installation portion 3212 from the outside of the heat exchanger installation portion 3212. The installation partition wall 3211 may protrude from a side surface of the duct body 321 and support a front side of the evaporator 341.

In addition, the duct body 321 may extend rearward with a width expanded based on the installation partition wall 3211.

As a result, a width of the heat exchanger installation portion 3212 may be greater than half the width of the base 310. In addition, a width of the circulation duct 320 may be greater than half the width of the base 310.

The width of the condenser 343 and the width of the evaporator 341 may also be greater than half the total width of the base 310.

As described above, when the widths of the condenser 343 and the evaporator 341 are secured, a heat exchange capacity may be sufficiently secured.

In addition, the fan housing 350 may be disposed to overlap the condenser 343 or the evaporator 341 in the front and rear direction. Therefore, air that has passed through the evaporator 341 and the condenser 343 may be introduced into the fan housing 350 without bending of the flow channel. In other words, air introduced into the circulation duct 320 may minimize the flow loss because the flow channel is not bent during the process of flowing to the fan housing.

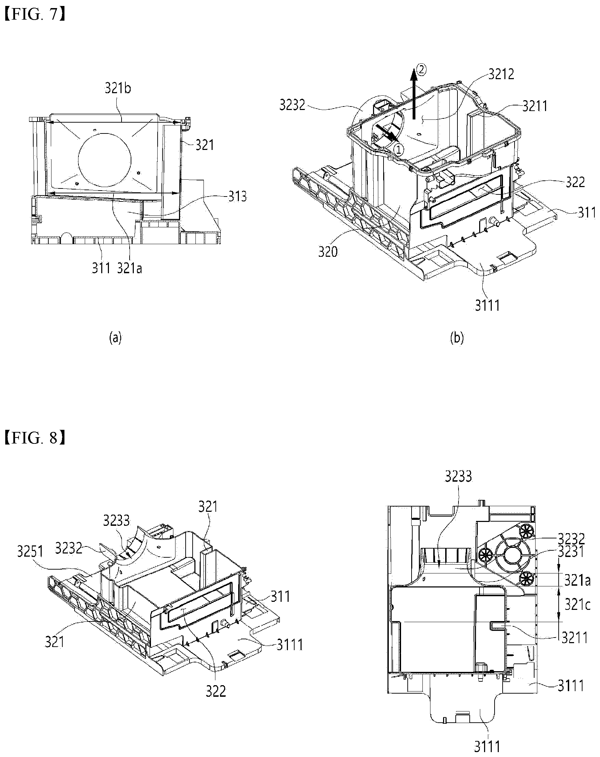

FIG. 7 illustrates a shape of a circulation duct of a laundry treating apparatus of the present disclosure.

The base 310 may have the base bottom 311 and the circulation duct 320 formed integrally with each other via mold injection.

A mold forming an inner surface of the duct body 321 may be removed by being withdrawn upward from inside of the duct body 321. In this regard, to facilitate the withdrawal of the mold, a wall surface of the duct body 321 may be inclined at a predetermined angle with respect to the removal direction of the mold.

A width of a bottom surface 321a of the duct body 321 may be greater than a width of a top surface 321b of the duct body 321.

Specifically, a distance between the wall surfaces of the duct body 321 facing each other may increase as it gets farther from the base bottom 311. A distance between a left side surface and a right side surface of the circulation duct facing each other may increase along the withdrawal direction of the mold. Accordingly, the removal of the mold may become easier.

In one example, the air discharger 323 may include an air extension pipe 3231 that extends from a rear side of the duct body 321 such that a diameter or a width thereof decreases, and an air discharge pipe 3232 that extends from the air extension pipe 3231 in a pipe shape with a uniform diameter to define a hollow 3233 therein. The air extension pipe 3231 may perform a function of a nozzle and thus increase a speed of discharged air.

In addition, a mold forming the air discharger 323 may be removed as shown in the drawing above. The mold may be withdrawn forward from the inside of the air discharger 323 toward the inside of the circulation duct 320, and then removed toward the upper open surface of the circulation duct 320. The air discharger 323 may be formed in a structure that facilitates the withdrawal of the mold in such process.

FIG. 8 is a cross-sectional view of the circulation duct.

The installation partition wall 3211 may protrude inward from the inner wall of the duct body 321 or may be formed as an outer wall of the circulation duct is recessed inward.

The heat exchanger installation portion 3212 may be formed between the heat exchanger installation partition wall 3211 and the air discharger 323.

The mold forming the air discharger 323 may be removed by being withdrawn forward from the air discharger 323 and then being withdrawn upward. It is necessary to prevent the mold for forming the above air discharger 323 from interfering with the heat exchanger installation partition wall when it is withdrawn forward from the inside of the air discharger 323. To this end, a design value of the air discharger 323 may be adjusted.

Specifically, when forming the air discharger 323, a mold for forming a front side and a mold for forming a rear side based on a parting line 3233 of the air discharger 323 in the drawing may be separately disposed. Accordingly, removal directions of the molds may also be different from each other. The mold for forming the portion located forward of the parting line of the air discharger 323 may be withdrawn forward, and the mold for forming the portion located rearward of the parting line of the air discharger 323 may be withdrawn rearward.

That is, to prevent the mold withdrawn forward from interfering with the heat exchanger installation partition wall during the withdrawal process, a distance 1 323a may be smaller than a distance 2 321c in the drawing. The distance 1 323a may mean a distance between the parting line of the air discharger 323 and a front end of the air discharger 323. In addition, the distance 1 323a may mean a distance between the parting line of the air discharger 323 and a rear opening of the circulation duct. The distance 2 321c may mean a distance between the front end of the air discharger 323 and the heat exchanger installation partition wall. In addition, the distance 2 321c may mean a distance between the rear opening of the circulation duct and the heat exchanger installation partition wall 3211.

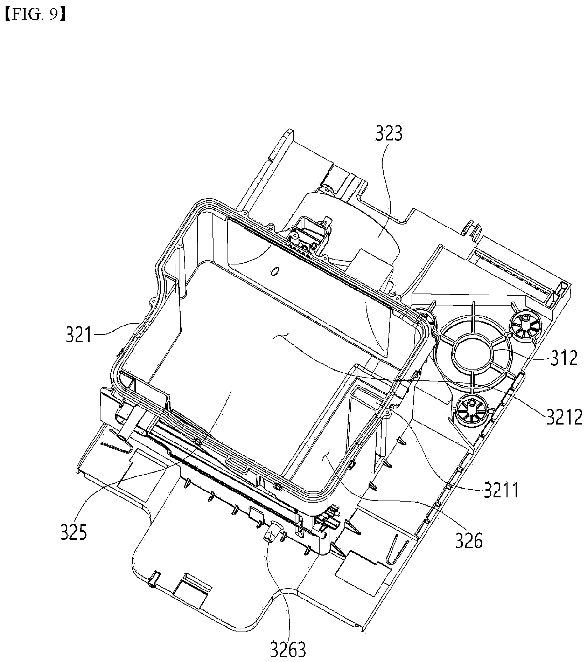

FIG. 9 shows a structure of a reservoir of a laundry treating apparatus of the present disclosure in detail.

In the laundry treating apparatus of the present disclosure, when the compressor 342 and the blower fan 352 are operated, air supplied from the outside of the cabinet 100 and air supplied from the inner casing 200 are cooled while passing through the evaporator 341, and water vapor contained in air is condensed.

Water condensed in the evaporator 341 may accumulate on the bottom surface of the circulation duct 320.

The laundry treating apparatus of the present disclosure may include a reservoir 326 formed as a portion of the bottom surface of the duct body 321 is recessed to collect condensate condensed in the evaporator 341.

The reservoir 326 is a space defined as the portion of the bottom surface of the duct body 321 is recessed, and is also able to form one side surface of the controller installation portion 313.

The reservoir 326 may be formed to be recessed downward from the bottom surface of the circulation duct 320.

The reservoir 326 may be formed integrally with the circulation duct 320. The reservoir 326 may be formed by forming the portion of the bottom surface of the circulation duct 320 to be recessed when injection-molding the circulation duct 320 onto the base 310.

At least a portion of a top surface of the reservoir 326 may be disposed parallel to the heat exchanger installation portion 313.

The base 310 may include a drain pipe 3263 that discharges water collected in the reservoir 326 to the outside.

The drain pipe 3263 may protrude from a lower portion of the reservoir 326 to the outside of the circulation duct 320. The drain pipe 3263 may discharge water stored in the reservoir to the outside of the base. As a result, water collected in the reservoir 326 may be prevented from rotting or flowing back to the bottom surface of the circulation duct 320.

The circulation duct 320 has the partition wall 3211 that extends from an inner surface of the duct body 321. The partition wall 3211 may protrude inward from the inner wall of the circulation duct 320 or may protrude inward as an outer wall of the circulation duct 320 is recessed inward. The partition wall 3211 may guide the locations where the heat exchangers 341 and 343 are installed, and may prevent air entering the heat exchanger from bypassing the heat exchanger.

The partition wall 3211 may be disposed in the reservoir 326.

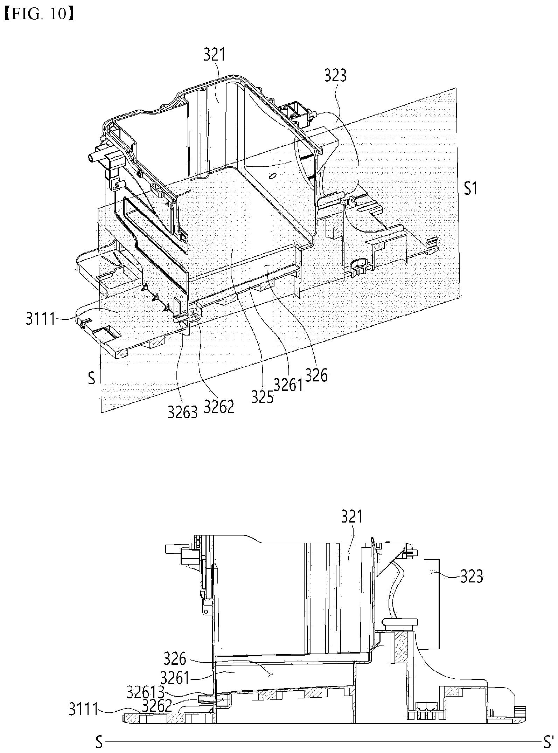

FIG. 10 is a cross-sectional view (S-S') of a circulation duct cut in a height direction.

The reservoir 326 may include a bottom surface 3261 where water accumulates, and a recessed portion 3262 that is recessed further downward from the bottom surface 3261. The drain pipe 3263 may be disposed on an outer surface of the circulation duct 320 at a location corresponding to the recessed portion 3262. As a result, the drain pipe 3263 may be disposed at a portion of the reservoir 326 with the lowest water level. Water collected in the reservoir 326 may flow to the drain pipe 3263 by its own weight.

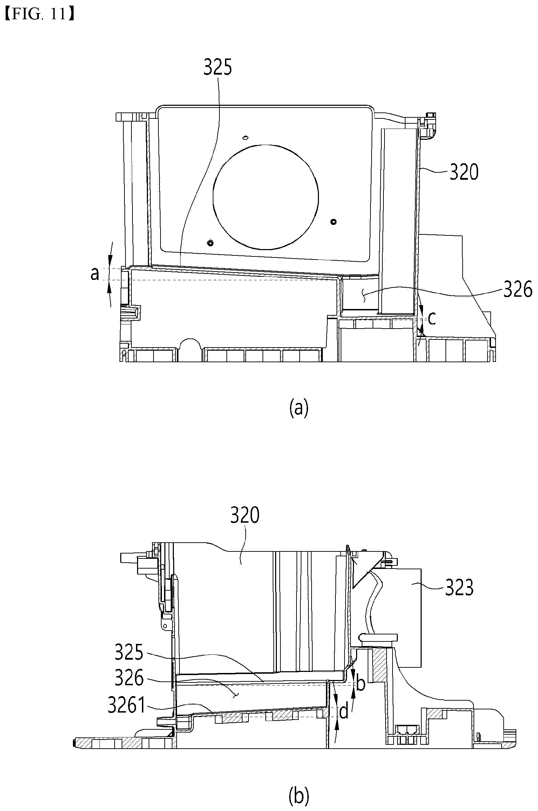

FIG. 11 shows an inclined structure related to a reservoir.

(a) in FIG. 11 shows a vertical cross-section parallel to the width direction of the base, and (b) in FIG. 11 shows a vertical cross-section parallel to the front and rear directions of the base.

The bottom surface of the circulation duct 320 and the bottom surface of the reservoir 326 may have a predetermined inclination.

In particular, a bottom surface 325 of the circulation duct may be inclined toward the reservoir 326, and the bottom surface 3261 of the reservoir may be inclined toward the drain pipe 3263.

The bottom surface 325 of the circulation duct may be disposed to be inclined at an angle 1 a toward the reservoir 326 based on the ground or the bottom surface of the base 310.

In addition, the bottom surface 325 of the circulation duct may be inclined downward in a forward direction toward the drain pipe 3263. The bottom surface 325 of the circulation duct may be disposed to be inclined forward at an angle 2 b based on the bottom surface of the base 310.

As a result, water condensed on the bottom surface of the circulation duct may flow forward and toward the reservoir 326.

In one example, the bottom surface 3261 of the reservoir may also have a predetermined inclination.

The drain pipe 3263 may be disposed to be biased on an inner surface of the reservoir 326 rather than on the outer surface thereof.

The bottom surface 3261 of the reservoir may have a downward inclination toward the inside of the circulation duct 320 based on the bottom surface of the base 310.

The bottom surface 3261 of the reservoir may be inclined at an angle 3 c based on the bottom surface of the base 310, and the bottom surface 3261 of the reservoir may have an inclination direction opposite to the inclination of the bottom surface 325 of the circulation duct.

The angle 3 c may be an angle of downward inclination in a direction away from the partition wall 3211.

The bottom surface 3261 of the reservoir may have a downward inclination toward the drain pipe 3263.

The bottom surface 3261 of the reservoir 326 may have a downward inclination in a forward direction at an angle 4 d based on the base 310.

The above-described angles 1 to 4 may be determined by a mold during a molding process of the base 310. The angles 1 to 4 may be determined during a molding process of the circulation duct 320 or the reservoir 326. The angles 2 b and 4 d may form inclinations in the same direction.

A mold for molding the reservoir 326 may be removed by being withdrawn upward. In this regard, side walls of the reservoir 326 may be tapered to facilitate the removal of the mold. Specifically, a cross-sectional area of the reservoir 326 may increase based on the withdrawal direction of the mold. In other words, a perimeter of the top surface of the reservoir 326 may be greater than a perimeter of the bottom surface of the reservoir 326.

A front surface of the reservoir 326 may be inclined forward in an upward direction. A rear surface of the reservoir 326 may be inclined rearward in the upward direction. Left and right side surfaces of the reservoir 326 may be inclined leftward and rightward in the upward direction, respectively.

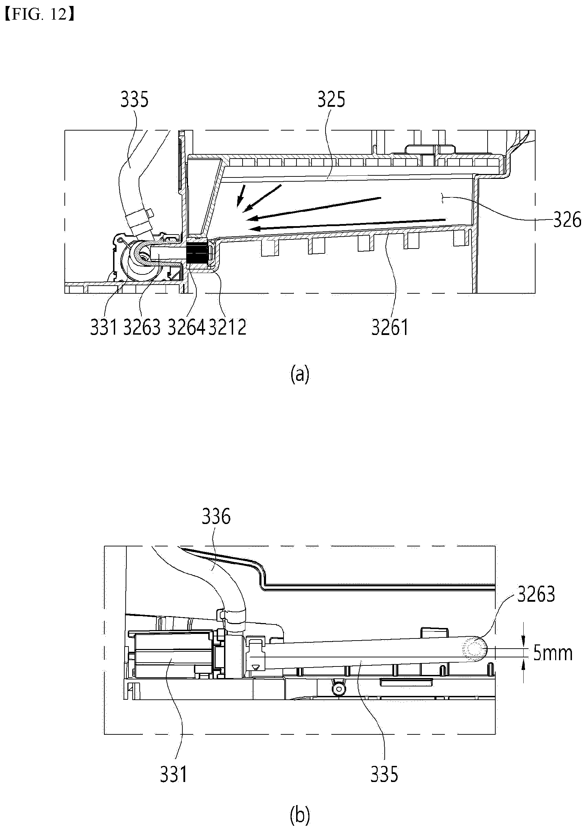

FIG. 12 shows structures of the reservoir and a residual water treater.

(a) in FIG. 12 is a cross-sectional view in a front and rear direction of the reservoir, and (b) in FIG. 12 shows a front portion of the bottom surface of the circulation duct 320.

The reservoir 326 may be disposed such that the bottom surface 3261 is inclined downward in a forward direction, and the bottom surface 325 of the circulation duct 320 may also be inclined downward in the forward direction.

A filter 3264 may be disposed in the recessed portion 3262 to prevent foreign substances from being discharged outside the drain pipe 3263.

The laundry treating apparatus of the present disclosure may include a residual water treater 330 that collects water collected in the reservoir 326 into the drainage tank 40.

The residual water treater 330 may include a drainage pump 331 that discharges water collected in the reservoir 326 into the drainage tank 40. The drain pipe 3263 and the drainage pump 331 may be connected to each other via a first drainage hose 3351, and water discharged from the drainage pump 331 may flow along a second drainage hose 3352.

The drain pipe 3263 may be disposed upward of the drainage pump 331. As a result, water collected in the reservoir 326 by its own weight may be collected to the drainage pump 331.

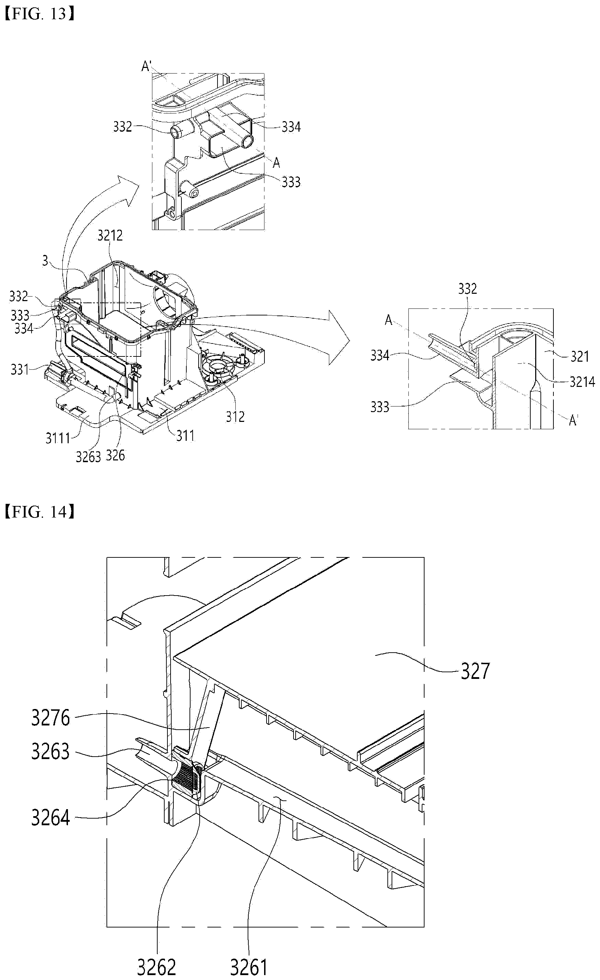

FIG. 13 shows an embodiment of a residual water treater of a laundry treating apparatus of the present disclosure.

Because condensate collected in the reservoir 326 should be collected in the drainage tank 40, the laundry treating apparatus of the present disclosure may include the residual water treater 330 that collects the condensate in the drainage tank 40.

In one example, because the drainage tank 40 is disposed in front of the duct body 321, it may be advantageous for the residual water treater 330 to also be disposed in front of the duct body 321.

In one example, the residual water treater 330 may install some of components connecting the drainage pump 331 with the drainage tank 40 on the base 310. Accordingly, when the drainage tank 40 is full or condensate flows back to the drainage tank 40, the condensate may be transferred back into the base 310 and circulated to the reservoir 326. Accordingly, the condensate may be prevented from leaking out of the base 310.

The reservoir 326 may include the drain pipe 3263 that discharges the condensate out of the reservoir 326. The drain pipe 3263 may extend forward from the base 310.

The residual water treater 330 may include the drainage pump 331 that provides power to transfer water discharged into the drain pipe 3263 to the drainage tank 40.

The residual water treater 330 may include an inlet pipe 332 that extends from one side of the circulation duct and is in communication with the drainage pump 331.

The residual water treater 330 may include a discharge pipe 334 that is in communication with the inlet pipe 332 and delivers the condensate to the drainage tank 40, and the discharge pipe 334 may be formed integrally with the base 310.

The residual water treater 330 may further include a guide pipe 333 disposed below the discharge pipe. The guide pipe 333 may allow the drainage tank 40 and the circulation duct 320 to be in communication with each other. The guide pipe 333 may guide water inside the drainage tank back into the circulation duct 320 when a water level of the drainage tank 40 is equal to or higher than a preset water level.

Water guided to the circulation duct 320 may be recovered in the reservoir 326 again and guided back to the drainage tank 40 via the residual water treater 330.

As a result, even when the drainage tank 40 is full, the condensate flows into the circulation duct 320 via the guide pipe 333, thereby preventing water in the drainage tank 40 from overflowing.

Recovery flow channel configuration drawing deleted

FIG. 14 shows an embodiment of a water cover.

The laundry treating apparatus of the present disclosure may further include a water cover 327 that is seated on the bottom surface of the circulation duct 320. The water cover 327 may support at least one of the evaporator 341 and the condenser 343, may block water condensed in the evaporator 341 from flowing to the condenser 343, and guide water to the reservoir 326.

The water cover may prevent the bottom surface of the circulation duct from being exposed to the outside. In addition, the water cover may form the support surface on which the evaporator and the condenser are supported. The water cover may support the evaporator and the condenser so as to be spaced apart from the bottom surface of the circulation duct. The water cover may shield the top surface of the reservoir. That is, the water cover may perform a cover function of the reservoir.

The water cover 327 may also shield an upper portion of the reservoir 326. As a result, air introduced into the circulation duct 320 may be blocked from being obstructed by a step between the reservoir 326 and the circulation duct 320.

The water cover 327 may include a water body 3271 having a plate shape and supporting at least one of the evaporator 341 and the condenser 343, and a support rib 3276 extending downward from the water body 3271 to maintain a vertical level or an inclination of the water body 3271.

One of the support ribs 3276 may be supported by the recessed portion 3262 or the filter 3264. As a result, the support rib 3276 may directly guide water flowing along the water body 3271 to the drain pipe 3263.

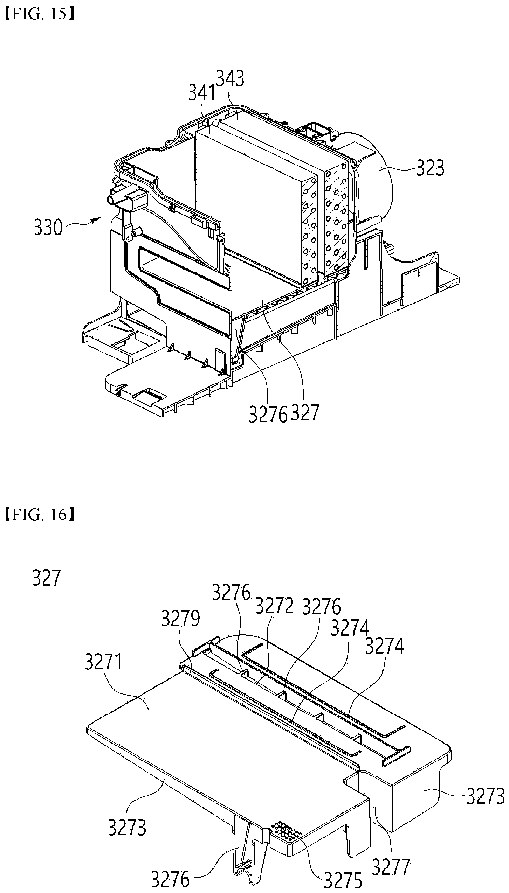

FIG. 15 shows a state in which the water cover is installed in the circulation duct.

The water cover 327 may be formed in a plate shape that shields at least a portion of the bottom surface of the circulation duct 320.

The water cover 327 may block the reservoir 326 from being exposed to an area facing the inlet 362 or an area into which outside air is introduced.

The water cover 327 may support lower ends of the evaporator 341 and the condenser 343. Even when the bottom surface of the circulation duct 320 is disposed to be inclined because of the water cover 327, the evaporator 341 and the condenser 343 may be disposed at the same vertical level.

In addition, the water cover 327 may prevent locations of the evaporator 341 and the condenser 343 from changing.

The water body 3271 of the water cover 327 may be disposed with an inclination to be parallel to the base 310. This prevents air introduced into the evaporator 341 from receiving unnecessary inclination resistance.

FIG. 16 shows a detailed structure of a water cover.

The water cover may include the water body 3271 positioned upward of the bottom surface of the circulation duct or the bottom surface of the reservoir. The water body 3271 may prevent the bottom surface 325 of the circulation duct or the bottom surface 3261 of the reservoir from being exposed to the outside.

The water cover 327 may include a seating rib 3274 protruding upward from the water body 3271. The seating rib 3274 may fix at least one of the evaporator 341 and the condenser 343, and may also maintain a gap between the evaporator 341 and the condenser 343.

The water cover 327 may include a through-hole 3272 extending through the water body 3271. The through-hole 3272 may be defined between the evaporator 341 and the condenser 343. Thus, water condensed in the evaporator 341 may be guided downward of the water cover.

The water cover 327 may further include a water outlet 3275 extending through the water body 3271 and defined to be spaced apart from the through-hole 3272. The water outlet 3275 may be defined in an area facing the reservoir 326.

The water outlet 3275 may discharge water flowing along the top surface of the water body 3271 to the reservoir 326.

In addition, the water outlet 3275 may also guide water overflowing from the drainage tank 40 to the reservoir 326.

The water cover 327 may include a spacing rib 3273 supported on the bottom surface of the circulation duct 320 from the water body 3271. The spacing rib 3273 may have an inclination corresponding to the inclination of the bottom surface of the circulation duct 320, and may be in contact with the bottom surface of the circulation duct 320, thereby blocking the air from flowing between the water body 3271 and the bottom surface of the circulation duct 320.

In one example, the spacing rib 3273 may be disposed along a periphery of the water body 3271.

In one example, the water cover 327 may further include an avoidance portion 3277 that prevents interference with the partition wall 3211 of the circulation duct. The avoidance portion 3277 may be formed to be recessed from a side surface of the water body 3271. The avoidance portion 3277 may have a shape corresponding to a shape of the partition wall.

The water cover 327 may include a discharge rib 3276 supported by the reservoir 326. The discharge rib 3276 may be formed in a shape that does not shield the drain pipe 3263.

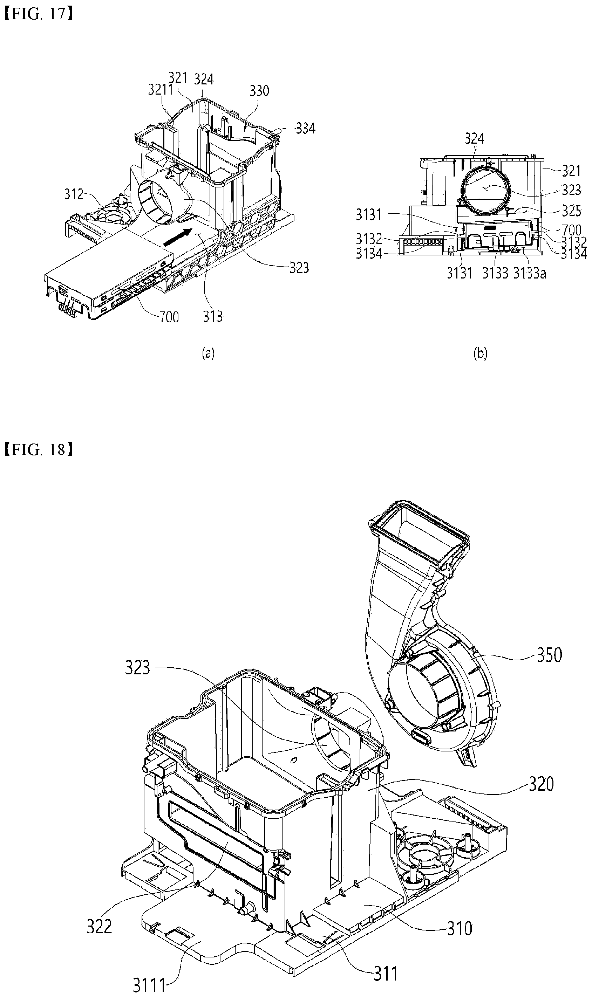

FIG. 17 shows a controller installation portion structure disposed on a base of a laundry treating apparatus of the present disclosure.
(a) in FIG. 17 shows an embodiment in which the controller 700 is installed in the controller installation portion 313.

The controller 700 may control all devices necessary for the laundry treating apparatus of the present disclosure to perform an arbitrary course of performing the refreshing cycle of the laundry. The controller 700 may be equipped as a PCB board, but may not be limited thereto and may be equipped as various devices for control.

The controller 700 may be inserted into and seated in the controller installation portion 313.

The controller installation portion 313 may be disposed downward of the circulation duct 320.

The bottom surface of the circulation duct 320 may form a top surface of the controller installation portion 313. The controller installation portion 313 may be disposed downward of the air discharger 323.

The controller installation portion 313 may be formed integrally with the base bottom 311. The controller installation portion 313 may be defined as a recessed space beneath the circulation duct during the process of forming the circulation duct 320 on the base 310.

The controller 700 may be introduced in a sliding manner in the forward direction into the controller installation portion 313.

A bracket 3131 may be further disposed on a surface of the controller 700 to surround the controller. The brackets 3131 may be disposed top and bottom surfaces of the controller to prevent the foreign substances from entering the controller.

In addition, the bracket 3131 may prevent damage to a circuit board inside the controller 700 resulted from heat or vibration transmitted to the controller 700. The bracket 3131 may be made of a metal material.

(b) in FIG. 17 shows a state in which a controller is installed in a controller installation portion.

As shown in the drawing, the controller 700 may be installed at a predetermined angle with the base bottom 311.

For example, the controller 700 may be disposed to be inclined toward the reservoir 326. Accordingly, when water leaks onto the top surface of the controller 700, the water may quickly escape the controller 700, and the bottom surface of the circulation duct 320 may be formed to be inclined toward the reservoir 326.

The controller 700 may include a supporter 3132 that is formed to protrude laterally.

The controller installation portion 313 may include a rib 3134 that protrudes from each of both side surfaces of the installation portion. The supporter 3132 of the controller may be disposed on the rib 3134.

The supporter 3132 of the controller may support a load of an entirety of the controller 700. When the supporter 3132 of the controller is supported on the rib 3134, the controller 700 may be spaced apart from the base bottom 311 by a predetermined distance.

The rib 3134 may be formed integrally with the base 310. The rib 3134 may be formed together with the base 310 when the base 310 is injection-molded, and may be formed integrally with the base bottom 311, the circulation duct 320, and the like.

A protrusion 3133 may be formed to protrude on a front surface of the controller 700. In addition, a guide protruding rearward may be disposed on an inner surface of the controller installation portion 313. The protrusion may be coupled with the guide. The protrusion may be inserted into the guide. When inserting the controller into the controller installation portion, the controller may be aligned at a correct location by coupling the protrusion to the guide.

In addition, locations of both side surfaces of the controller may be determined in the manner in which the supporter is seated on the rib as described above. Using such coupling process, the controller may be coupled at the correct location of the controller installation portion without a separate fastening member.

FIG. 18 shows a structure of an air discharger 323 of a laundry treating apparatus of the present disclosure.

The base 310 may include the air discharger 323 that discharges treated air toward the fan housing.

The air discharger 323 may allow the inside of the circulation duct 320 or the duct body 321 to be in communication with the fan housing 350. The air discharger 323 may be formed in a bell mouth shape. The bell mouth shape may reduce the flow loss of air and improve an air circulation efficiency.

The air discharge pipe 3232 of the air discharger 323 may be formed in a pipe shape, and in the mold removal process, based on the parting line 3233, the mold disposed forward of the parting line 3233 may be withdrawn forward, and the mold disposed rearward of the parting line 3233 may be withdrawn rearward.

The fan installation portion 350 may be supported by being coupled to the air discharge pipe 3232. The fan housing 351 may have a coupling hole coupled with an outer circumferential surface of the air discharge pipe 3232, and the blower fan 353 may be disposed in the coupling hole.

The fan housing 351 may include the exhaust duct 352 extending from an outer circumferential surface or an outer side of the blower fan 353 to the exhaust hole 232.

The fan housing 351 and the exhaust duct 352 may form therein a flow channel that accommodates the blower fan 353 therein and allows air to flow.

A motor that rotates the blower fan 353 may be supported by being coupled to the outer side of the fan housing 351.

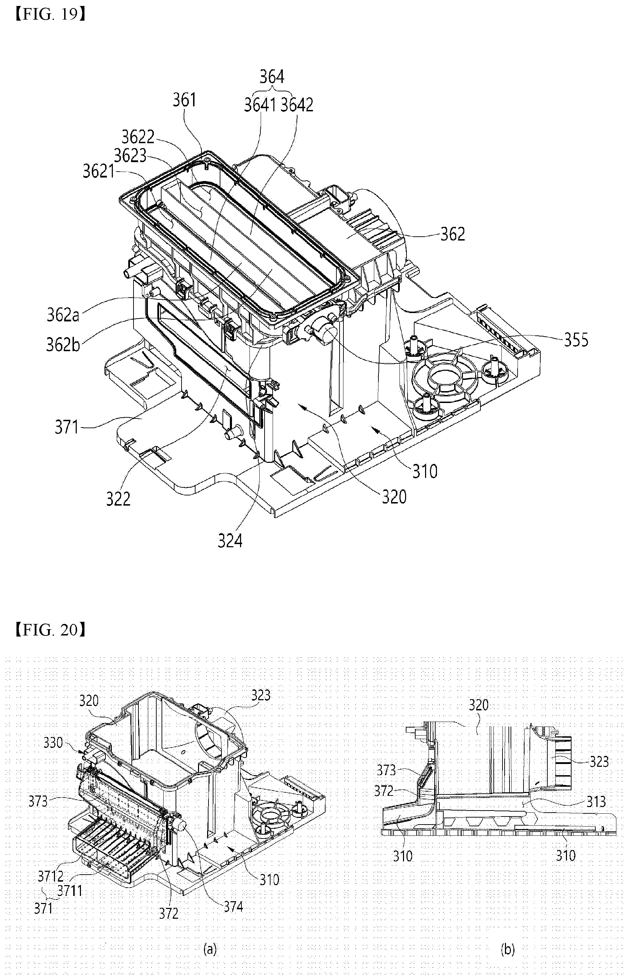

FIG. 19 shows a structure of a base cover of a laundry treating apparatus of the present disclosure.

The base cover 360 may be coupled to the top surface of the circulation duct 320 to prevent the inside of the circulation duct 320 from being exposed.

The base cover 360 may include an inflow body 361 coupled to the top surface of the circulation duct 320 to allow the inner casing 200 and the circulation duct 320 to be in communication with each other, and a shielding body 363 extending from the inflow body 361 to shield the circulation duct 320.

The inflow body 361 may be formed in a duct shape to allow the inflow hole 231 of the inner casing and the inside of the circulation duct 320 to be in communication with each other. The inflow body 361 may protrude higher than the shielding body 363.

The inflow body 361 may be disposed forward of the evaporator 341 so as not to face the evaporator 341 and the condenser 343, and may be disposed forward of the partition wall 3211.

The inflow body 361 may serve as an inflow duct that moves air of the inner casing 200 to the circulation duct 320.

The inflow body 361 may include the inlet 362 therein through which air of the inner casing 200 may pass.

Specifically, the base cover 360 may include a first rib 362a extending along a width direction of the inflow body 361 and a second rib 362b extending along the width direction of the inflow body 361 and spaced rearwardly apart from the first rib 362a.

The first rib 362a and the second rib 362b may be disposed to be in parallel with each other. The first rib 362a and the second rib 362b may be formed in a plate shape extending in a vertical direction, and a height thereof may correspond to a height of the inflow body 361.

A front edge of the inflow body 361 and the first rib 362a may form a first inlet 3621, the first rib 362a and the second rib 362b may form a second inlet 3622, and the second rib 362b and a rear edge of the inflow body 361 may form a third inlet 3623.

The first inlet 3621 and the third inlet 3623 may have the same area size, and the second inlet 3622 may have a smaller area size than the first inlet 3621 and the third inlet 3623.

The base cover 360 may include a damper assembly 364 that opens and closes the inlet 362, and a driver 365 coupled to the damper 364 to control the opening and closing of the damper assembly 364.

The damper assembly 364 may include a first damper 3641 that opens and closes the first inlet 3621, and a second damper 3642 that opens and closes the third inlet 3623.

The first damper 3641 may be formed in a plate shape with an area size corresponding to that of the first inlet 3621, and may be rotatably coupled to both side surfaces of the inflow body 361 inside the first inlet 3621.

The second damper 3642 may be formed in a plate shape with an area size corresponding to that of the third inlet 3623, and may be rotatably coupled to both side surfaces of the inflow body 361 inside the third inlet 3623.

The second inlet 3622 may include a blocking filter 366 that allows air to pass therethrough, but is able to filter out the foreign substances such as fine dust and lint.

The blocking filter 366 may be inserted into the second inlet 3622 to partition the first inlet 3621 and the third inlet 3623 from each other. The blocking filter 366 may be disposed in the second inlet 3622 to extend so as to be in contact with the bottom surface of the circulation duct 320.

The blocking filter 366 may be equipped as a filter that may filter even moisture. For example, the blocking filter 366 may be equipped as a HEPA filter or the like.

In one example, when the blocking filter 366 is inserted into the second inlet 3622, a shielding member that shields the second inlet 3622 may be further coupled.

The driver 365 may include a motor that provides power to selectively rotate the first damper 364 and the second damper 365, and a plurality of gear members that may selectively rotate the first damper 364 and the second damper 365 while rotating in a state of being engaged with the motor.

The first inlet 3621 and the third inlet 3623 may be selectively opened by the driver 365.

Because of the driver 365, air contained within the inner casing 200 may be introduced into the circulation duct 320 along the first inlet 3621 or may be introduced into the circulation duct 320 along the third inlet 3623.

In one example, the driver 365 may control the first damper 3641 and the second damper 3642 to open both the first inlet 3621 and the third inlet 3623, and may control the first damper 3641 and the second damper 3642 to shield both the first inlet 3621 and the third inlet 3623.

The driver 365 may be formed with any structure as long as it may rotate the first damper 3641 and the second damper 3642. For example, the driver 365 may be formed with a combination of the motor, a driving gear that rotates by the motor, and a driven gear that is coupled to the first damper and the second damper and rotates by the rotation of the driving gear.

The base cover 360 may include the shielding body 363 that may extend from the inflow body 361 and shield the evaporator 341 and the condenser 343. The shielding body 363 may be formed in a plate shape.

The base cover 360 may be detachably coupled to the top surface of the circulation duct 320 via an inflow hook 3612 that extends from the bottom surface of the inflow body 361.

The circulation duct 320 may include a coupling portion that is detachably coupled to the inflow hook 3612.

FIG. 20 shows a structure of an outside air duct.

Referring to (a) in FIG. 20, the outside air duct 370 may be coupled to the base 310.

The outside air duct 370 may be in communication with the outside air intake portion 322.

The outside air duct 370 may include an outside air damper 373 that opens and closes the outside air intake portion 322, and an outside air driver 374 that rotates the outside air damper 373 to selectively open the outside air intake portion 322.

The outside air damper 373 may be formed in a plate shape that may seal the outside air intake portion 322, and may be rotatably coupled to both side surfaces of the outside air intake portion 322.

The outside air driver 374 may be equipped as an actuator that is coupled to the outside air duct 370 or the circulation duct 320 and rotates the outside air damper 373.

The outside air duct 370 may include an extension duct 372 that extends forward from the outside air intake portion 322, and an intake duct 371 that extends forward from the extension duct 372 and allows outside air to be introduced.

The intake duct 371 may extending from a lower portion of the extension duct 372, and may have the water supply tank 30 and the drainage tank 40 disposed thereon. The water supply tank 30 and the drainage tank 40 may be coupled to or seated on the intake duct 371.

The intake duct 371 may include an outside air port 3711 into which outside air is sucked and a partition rib 3712 that partitions the outside air port 3711 at one end or a free end thereof.

The outside air port 3711 may be disposed downward of the door 400 so as not to be shielded by the door 400.

The partition rib 3712 may partition the inside of the outside air port 3711 so as to block the foreign substances or a user's body from being inserted.

Referring to (b) in FIG. 20, when the outside air driver 374 rotates the outside air damper 373 to open the outside air intake portion 322, the intake duct 371 and the circulation duct 320 may be in communication with each other.

In this regard, when the blower fan 352 is operated, air outside the cabinet may be introduced into the circulation duct 320. When the compressor 342 is operated, the outside air may be dehumidified while passing through the circulation duct 320 and supplied into the inner casing 200.

The door 400 may further include an exhaust port that discharges air inside the inner casing 200 to the outside, and an exhaust damper that selectively opens and closes the exhaust port. The exhaust port may be disposed to face the accommodating space of the inner casing 200.

Thus, the dehumidified air may be discharged via the exhaust port.

In addition, the outside air may be filtered while passing through the blocking filter 366 and discharged back to the outside of the cabinet 100.

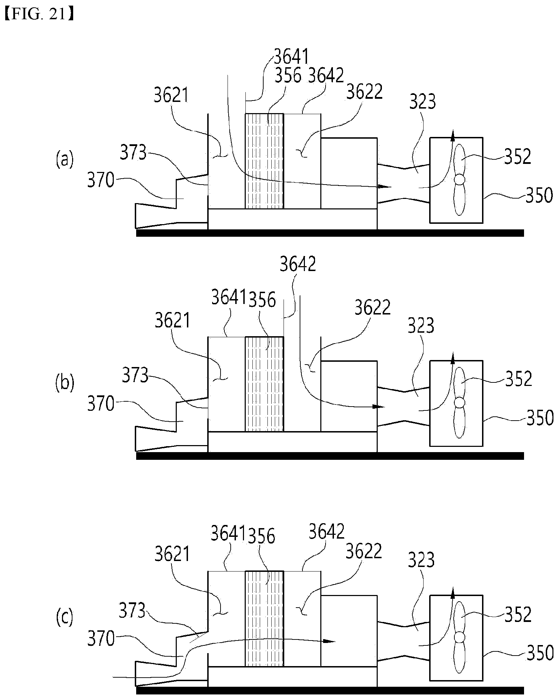

FIG. 21 shows a flow of air flowing through the circulation duct.

Referring to (a) in FIG. 21, the outside air damper 373 may be controlled to shield the outside air intake portion 322, the first damper 3641 may be controlled to open the first inlet 3621, and the second damper 3642 may be controlled to shield the third inlet 3623.

When the blower fan 352 is operated, air inside the inner casing 200 may be introduced into the first inlet 3621 and filtered while passing through the filter 366.

When the compressor 342 is operated, air that has passed through the filter 366 may be dehumidified and heated while passing through the evaporator 341 and the condenser 343.

Air that has also passed through the heat exchanger may be supplied into the inner casing 200 by passing through the fan installation portion 350.

This state may be a state in which steam is not supplied to the inner casing 200. This is because when steam is supplied to the inner casing 200, moisture will wet the filter 600, and a performance of the filter 366 will not be guaranteed.

As a result, when steam has not been supplied to the inner casing 200, before steam is supplied to the inner casing 200, or when humidity is lowered even after steam is supplied to the inner casing 200, air inside the inner casing 200 may pass through the first inlet 3641 and may filter out the foreign substances such as lint while passing through the filter 366.

Referring to (b) in FIG. 21, the outside air damper 373 may be controlled to shield the outside air intake portion 322, the first damper 3641 may be controlled to shield the first inlet 3621, and the second damper 3642 may be controlled to open the third inlet 3623.

When the blower fan 352 is operated, air inside the inner casing 200 may be introduced into the third inlet 3623. Because the third inlet 3623 is disposed downstream of the blocking filter 366, air introduced into the third inlet 3623 may not pass through the blocking filter 366.

When the compressor 342 is operated, air that has passed through the filter 366 may be dehumidified and heated while passing through the evaporator 341 and the condenser 343.

Air that has also passed through the heat exchanger may be supplied into the inner casing 200 by passing through the fan installation portion 350.

As a result, when steam has been supplied to the inner casing 200 or the humidity inside the inner casing 200 is very high, air of the inner casing 200 may be allowed to be introduced into the third inlet 3623 and blocked from being introduced into the first inlet 3621, thereby preventing the blocking filter 366 from being exposed to moisture.

Referring to (c) in FIG. 21, the outside air damper 373 may be controlled to open the outside air intake portion 322, the first damper 3641 may be controlled to shield the first inlet 3621, and the second damper 3642 may be controlled to shield the third inlet 3623.

When the blower fan 352 is operated, air inside the inner casing 200 may be blocked from flowing into the inlet 362, and only outside air of the cabinet 100 may flow into the circulation duct 320 and pass through the blocking filter 366. As a result, the foreign substances such as fine dust contained in outside air may be filtered by the blocking filter 366.

When the compressor 342 is operated, air that has passed through the filter 366 may be dehumidified while passing through the evaporator 341 and the condenser 343.

Air that has also passed through the heat exchanger may be supplied into the inner casing 200 by passing through the fan installation portion 350 to supply fresh hot air to the laundry.

In this regard, when an apparatus for discharging air inside the inner casing 200 to the outside is disposed in the door 400, air outside the cabinet may be discharged in a purified and dehumidified state while passing through the blocking filter 366 and the heat supply 340.

As a result, the laundry treating apparatus of the present disclosure may determine flow directions of air inside the inner casing 200 and air outside the cabinet as the controller 700 controls the outside air driver 374 and the inlet driver 365.

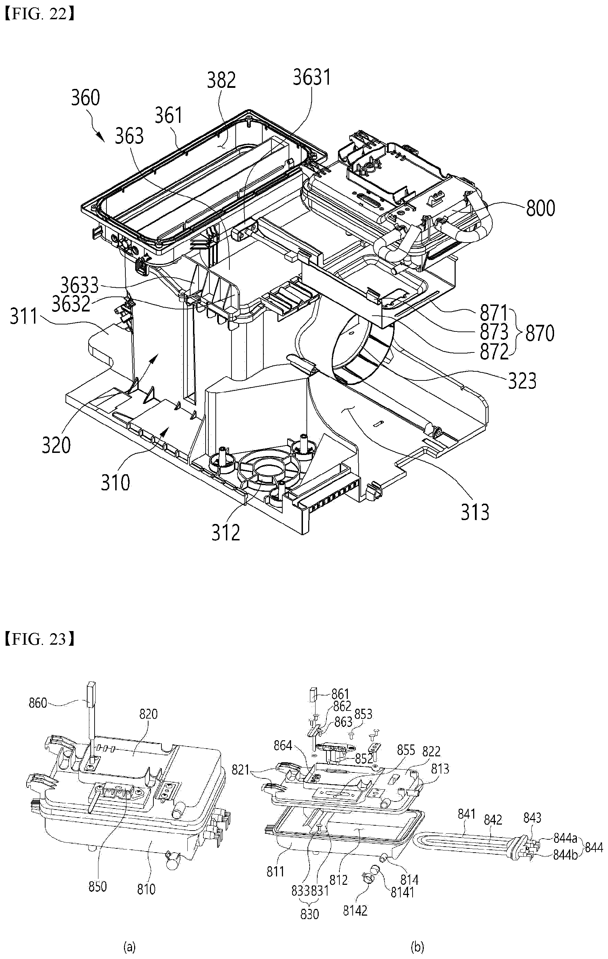

FIG. 22 shows an installation structure of a steam supply.

The steam supply 800 may be supported by being seated on the base cover 360.

The steam supply 800 may include a steam generator 810 that is seated on the base cover 360 and stores water that generates steam.

The steam supply 800 may further include an installation bracket 870 that may fix the steam generator 810 to the base cover 360.

The installation bracket 870 may be coupled to the base cover 360 to fix the steam generator 810.

The installation bracket 870 may include a lower panel 871 that supports a bottom surface of the steam generator 810 and side panels 872 that support both side surfaces of the steam generator 810 on the lower panel 871.

The installation bracket 870 may further include one or more fixing clips 873 that extend from the side panels 872 to prevent the steam generator 810 from being detached.

The fixing clip 873 may be detachably disposed on a top surface or the side surface of the steam generator 810.

The compressor 342 may be disposed downward of the steam supply 800.

The installation bracket 870 may block heat generated from the compressor or heat generated from a refrigerant compressed in the compressor from being transferred to the steam supply 800.

The installation bracket 870 may also block fire from being transferred to the steam supply 800 in the event of fire occurring in the compressor 342.

In one example, the base cover 360 may include a fastener 3631 disposed on the shielding body 363 and detachably coupled with the steam supply 800. The fastener 3631 may have a structure detachably coupled with a protrusion protruding from a lower portion of the steam generator 810.

As a result, even when a large amount of water is contained inside the steam generator 810, the steam generator 810 may be stably seated on the base cover 360.

In addition, because the steam generator 810 is positioned upward of the circulation duct 320 and thus a distance thereof to the inner casing 200 becomes smaller, condensation of steam generated in the steam generator 810 before reaching the inner casing 200 may be minimized.

FIG. 23 shows a detailed structure of the steam supply.

Referring to (a) in FIG. 23, the steam supply 800 may include the steam generator 810 that may receive and store water to generate steam, and a heater assembly 840 that is accommodated in the steam generator 810 and heats the water to generate steam.

The steam generator 810 may be formed in a casing shape that defines a space for accommodating the heater assembly 840.

For example, the steam generator 810 may be formed in a casing shape with an open top, and may accommodate the heater assembly 840 therein.

The steam supply 800 may further include a casing cover 820 that is coupled to the steam generator 810 to prevent the heater assembly 840 from being exposed to the outside and prevent water from leaking.

The casing cover 820 may be installed with a water level sensor 850 that senses a water level of the steam generator 810 and a steam sensor 860 that senses a temperature inside the steam generator 810 or senses whether steam is generated inside the steam generator 810.

Referring to (b) in FIG. 23, the steam generator 810 may include a casing body 811 that provides a space for storing water and accommodating the heater assembly 840 therein.

The casing body 811 may be formed in a shape with an open top, so that various components may be easily installed inside the casing body 811.

The casing body 811 may include a heater insertion hole 8111 extending through one side thereof and through which the heater assembly 840 may be inserted or withdrawn.

The casing body 811 may include a recovery pipe 814 that receives water to generate steam therein.

The recovery pipe 814 may discharge water accommodated inside the casing body 811 to the outside.

The recovery pipe 814 may be kept closed by a shielding plug 8141 so as to be opened only when removing residual water inside the steam generator 810, and may include a shielding clip 8142 that keeps the shielding plug 8141 coupled to the recovery pipe 814 such that the shielding plug 8141 is prevented from being removed arbitrarily.

Therefore, when repairing the steam generator 800 or preventing freezing or the like of the steam generator 800, water inside the steam generator 800 may be discharged via the recovery pipe 814.

In one example, the recovery pipe 814 may receive water from the water supply tank 30. Details will be described later.

In one example, a heater fixer 830 that may support or fix the heater assembly 840 may be installed inside the casing body 811. The heater fixer 830 may include a fixing clip 831 that fixes the heater assembly 840, and a clip fastening member 833 that fixes the fixing clip 831 to the casing body 811.

The fixing clip 831 may accommodate or surround at least a portion of the heater assembly 840.

In one example, the steam supply 800 may include a water supply pipe 815 that receives water. The water supply pipe 815 may be in communication with the water supply tank 30 to receive water.

The water supply pipe 815 may be disposed on the casing cover 820 or may be disposed at an upper portion of the steam generator 810. As a result, water may be prevented from flowing back via the water supply pipe 815.

The steam supply 800 may include a steam pipe 813 that discharges steam generated by the operation of the heater assembly 840 to the outside. The steam pipe 813 may also be disposed at an upper portion of the casing cover 820, so that water may be prevented from being discharged arbitrarily into the steam pipe 813.

The steam pipe 813 may be in communication with the steam hole 233 of the inner casing 200.

The casing cover 820 may include a water level sensor hole 854 in which the water level sensor may be installed.

The water level sensor 850 may include one or more contact protrusions 852 that are inserted into the water level sensor hole 854 and immersed in water to sense the water level, and a sensor body 851 that is coupled to the water level sensor hole 854 or supported by the casing cover 820 to maintain the contact protrusions 852 in a floating state inside the steam generator 810.

The sensor body 851 may be coupled to the casing cover 820 via a sensor fastening member 853.

In one example, the casing cover 820 may include an insertion hole 864 into which the steam sensor 860 may be installed. The steam sensor 860 may include a sensing device 861 that is inserted into the insertion hole 864 to sense whether steam is generated inside the steam generator 810, a support member 863 that fixes the sensing device 861 to the casing cover 820, and a coupling member 862 that couples the support member 863 to the casing cover 820.

The sensing device 861 may be equipped as a humidity sensor or a temperature sensor and may sense whether steam is generated inside the steam generator 810.

In one example, the casing cover 820 may include a cover hook 821 that extends forward to be coupled to the base cover 860.

In addition, a fixing protrusion 822 that may fix a lower portion of the inner casing 200 or a separate steam discharger 900 may also be disposed at a rear side of the casing cover 820.

The heater assembly 840 may be inserted into the heater insertion hole 8111, be accommodated in the steam generator 810, and receive power to heat water.

The heater assembly 840 may be equipped as a sheath heater or the like, and may be controlled by the controller 700 such that operation and stopping may be repeated.

The heater assembly 840 may include a first heater 841 that receives first power to heat water, and a second heater 842 that receives power greater than the first power to heat water.

As a result, the second heater 842 may heat a larger amount of water and generate more steam than the first heater 841.

The first heater 841 and the second heater 842 may consume maximum heater power allowed for the heater assembly 840 in the laundry treating apparatus in a shared manner. That is, when the first heater 841 consumes a portion of the maximum heater power, the second heater 842 may consume the remainder of the maximum heater power.

For example, when the general maximum heater power allowed for the heater assembly 840 is 1500W, the first heater 841 may consume 600W, and the second heater 842 may consume 880W. The remaining 20W may be allocated as a margin to account for an error or the like.

In one example, the heater assembly 840 may include three or more heaters. For example, the first heater 841, the second heater 842, and a third heater 843 may be included, and the first heater 841, the second heater 842, and the third heater 843 may consume the maximum heater power in the shared manner.

Hereinafter, a description will be made based on the heater assembly 840 being composed of the first heater 841 and the second heater 842.

Each of the first heater 841 and the second heater 842 may be formed as a U-shaped metal pipe.

The heater assembly 840 may include a heater sealer 843 that may fix the first heater 841 and the second heater 842 and seal the heater through-hole 8111, and may include a terminal assembly 844 that supplies current to the first heater 841 and the second heater 842.

The terminal assembly 844 may include a first terminal 844a that supplies current to the first heater 841 and a second terminal 844b that supplies current to the second heater 842.

The first heater 841 and the second heater 842 may be arranged at the same vertical level. Accordingly, the first heater 841 and the second heater 842 may heat water at the same water level to generate steam.

Therefore, the controller 700 may adjust an amount of steam generated and an amount of power consumed using both or selectively the first heater 841 and the second heater 842.

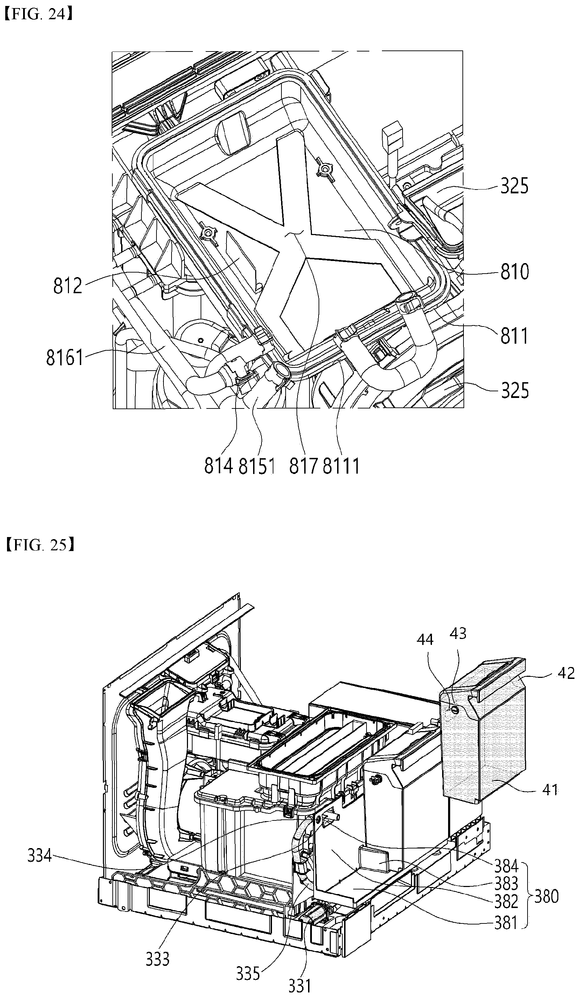

FIG. 24 shows inside of the steam generator.

The steam generator 810 may have a heating space 817 that stores water therein and accommodates the heater assembly 840 therein.

In addition, the steam generator 810 may receive water to generate steam via a water supply hose 8151 connected to the water supply pipe 815.

In one example, steam generated by operating the heater assembly 840 in the steam generator 810 may be discharged to the outside of the steam supply 800 via the steam discharge pipe 813 along a steam hose 8131.

The steam hose 8131 may be in communication with the steam hole 233 of the inner casing 200.

In one example, the steam generator 810 may include a separation partition wall 812 that may separate the heating space 817 and the water level sensor 850 from each other. That is, the separation partition wall 812 may separate the heater assembly 840 and the water level sensor 850 from each other, and may be disposed to be biased to one side of the casing body 811.

The water level sensor 850 may be disposed between the separation partition wall 812 and an inner surface of the casing body 811.

As a result, vibration of water occurring when the water boils may be prevented from being transmitted to the water level sensor 850, and heat generated in the heater assembly 840 may be prevented from being directly transmitted to the water level sensor 850.

FIG. 25 shows a steam supply in which the heater assembly is installed.

Referring to (a) in FIG. 25, the steam sensor 860 may be disposed upward of the heater assembly 840 to sense the temperature inside the steam generator 810 or the like. Thus, the steam sensor 860 may sense that steam is generated when the water reaches 100 degrees or a high temperature.

The heater assembly 840 may be supported by a support clip 832 to prevent the heater assembly 840 from coming into contact with the bottom surface of the steam generator 810 or the like.

In addition, an upper portion of the heat assembly 840 may be surrounded by the fixing clip 831 to prevent the water level at which the heater assembly 840 is disposed from changing.

Referring to (b) in FIG. 25, the heater sealer 844 of the heater assembly 840 may include a first support hole 8441 through which the first heater 841 extends and is supported and a second support hole 8442 through which the second heater 842 extends and is supported.

A first length L1 at which the first support hole 8441 is spaced apart from the bottom surface of the steam generator 810 and a second length L2 at which the second support hole 8442 is spaced apart from the bottom surface of the steam generator 810 may be equal to each other.

Even when diameters of the first support hole 8441 and the second support hole 8442 are different from each other, installation vertical levels thereof may be equal to each other.

Accordingly, the first heater 841 and the second heater 842 may heat water at the same water level to generate steam.

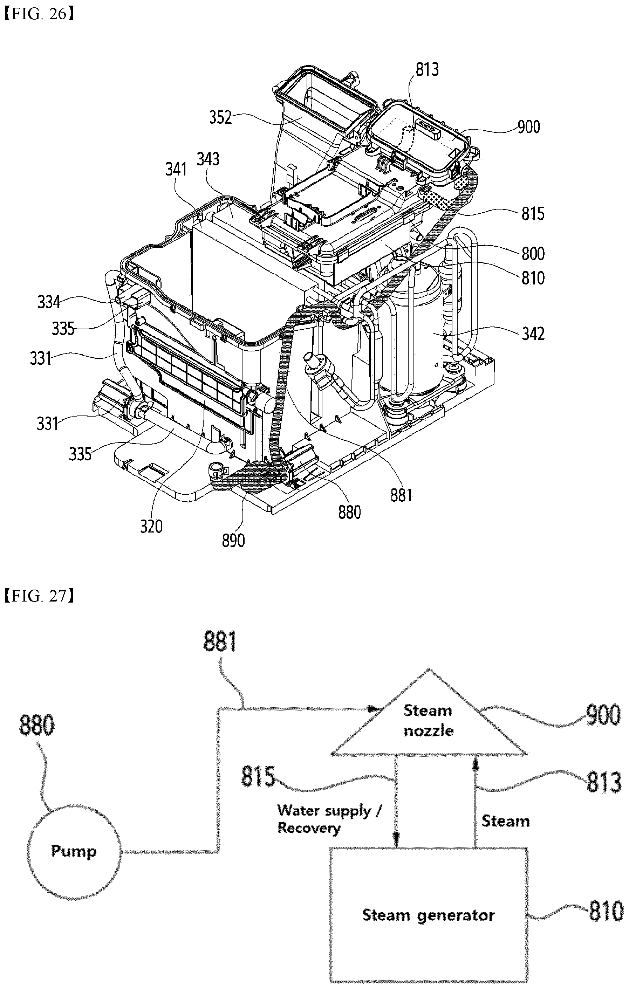

FIG. 26 shows a steam supply structure of a laundry treating apparatus of the present disclosure in detail.

Steam generated inside the steam generator 810 may be directly supplied into the inner casing 200 via the steam pipe 813.

However, when steam is directly supplied into the inner casing 200 via the steam pipe 813, there is a concern that water contained in the steam generator 810 may also be supplied into the inner casing 200.

In addition, there is a concern that water heated in the steam generator 810 may heat the bottom surface of the inner casing 200, causing thermal damage to the inner casing 200.

To prevent such problem, the steam supply 800 of the present disclosure may further include a steam nozzle 900 that receives steam generated from the steam generator 810 and supplies steam to the inner casing 200.

The steam nozzle 900 may be disposed to be spaced apart from the steam generator 810, and may receive only steam formed in the steam generator 810 and not receive water contained in the steam generator 810.

For example, the steam nozzle 900 may be disposed upward of the steam generator 810 and connected to the steam pipe 813. Accordingly, steam generated in the steam generator 810 may rise by a density difference and may be supplied to the steam nozzle 900 along the steam pipe 813, and water contained in the steam generator 810 may not be introduced into the steam pipe 813 or the steam nozzle 900 by gravity.

The steam nozzle 900 may be disposed between the bottom surface of the inner casing 200 and the steam generator 810, and may be in communication with the steam hole 233.

In one example, the steam supply 800 of the present disclosure receives water from the water supply tank 30 and generates steam.

To this end, the laundry treating apparatus of the present disclosure may further include a water supply pump 880 that supplies water accommodated in the water supply tank 30 to the steam supply 800.

The steam supply 800 may further include a supply pipe 890 that may guide water accommodated in the water supply tank 30 to the water supply pump 880.

In addition, the laundry treating apparatus of the present disclosure may further include the water supply pipe 815 that guides water discharged from the water supply pump 880 to the steam supply 800. The water supply pipe 815 may be formed in a shape of the water supply hose 8151 made of rubber or the like.

As a result, the water supply pump 880 may receive water from the supply pipe 890 connected to the water supply tank 30. In addition, the water supply pump 880 may discharge water to supply water to the steam supply 800 via a water supply pipe 881.

The steam supply 800 may be considered to include the water supply pump 880 that provides power to supply water accommodated in the water supply tank 30 to the steam generator 810.

The supply pipe 890 may be equipped as a hose connecting the water supply tank 30 with the water supply pump 880, and the water supply pipe 881 may be equipped as a hose coupled to the water supply pump 880.

In one example, the water supply pipe 815 may directly connect the water supply pump 880 with the steam generator 810. As a result, water supplied from the water supply pump 880 may be directly supplied to the steam generator 810, may be heated by the heater assembly 840, and generate steam. The steam may be supplied to the steam nozzle 900 via the steam pipe 813 and delivered to the inner casing 200.

However, as shown in FIG. 26, the water supply pipe 815 may directly connect the water supply pump 880 with the steam nozzle 900.

In other words, the laundry treating apparatus of the present disclosure may supply water accommodated in the water supply tank 30 to the steam nozzle 900 rather than to the steam generator 810. Water supplied from the water supply pump 880 may be directly supplied to the steam nozzle 900.

The water supply pump 880 may not be directly connected to the steam generator 810. The water supply pump 880 may be viewed as being in communication with the steam generator 810 via the steam nozzle 900.

The steam nozzle 900 may be supplied with water accommodated in the water supply tank 30 via the water supply pump 880. Water supplied to the steam nozzle 900 may be delivered to the steam generator 810.

Because the steam nozzle 900 is positioned upward of the steam generator 810, water supplied to the steam nozzle 900 may be automatically supplied to the steam generator 810.

As a result, the steam nozzle 900 may receive water accommodated in the water supply tank 30 via the water supply pump 880 and deliver water to the steam generator 810.

The steam supply 900 may further include the recovery pipe 815 connecting the steam nozzle 900 with the steam generator 810. The recovery pipe 815 may supply water supplied to the steam nozzle 900 and temporarily accommodated to the steam generator 810.

Therefore, the steam pipe 813 through which steam is introduced into the steam nozzle 900 and the recovery pipe 815 through which water is discharged from the steam nozzle 900 may be equipped as separate flow channels.

Accordingly, water supplied from the steam nozzle 900 may be blocked from interfering with the movement of steam supplied from the steam generator 810.

In one example, the recovery pipe 815 may be formed in a U-shape. That is, a partial area between both ends of the recovery pipe 815 may be disposed lower than said both ends. As a result, a certain amount of water, such as a water trap, may be accumulated in the recovery pipe 815, thereby preventing steam or water supplied from the steam generator 810 from being re-introduced via the recovery pipe 815.

The steam pipe 813 may include the steam hose 8131 connecting the steam nozzle 900 with the steam generator 810. The steam hose 8131 may be made of a rubber material.

The recovery pipe 815 may be disposed separately from the steam pipe 813, and may be disposed to be spaced apart from the steam pipe 813. The recovery pipe 815 may include a recovery hose 8151 connecting the steam nozzle 900 with the steam generator 810. The recovery hose 8151 may be made of a rubber material.

The steam generator 810 may receive and accommodate water supplied via the steam nozzle 900. Because the steam generator 810 is not directly connected to the water supply pump 880, there is no possibility that water introduced into the steam generator 810 will flow back to the water supply pump 880.

In addition, because the steam nozzle 900 is disposed upward of the steam generator 810 and the steam nozzle 900 is connected to the steam generator 810 via the recovery pipe 815, water supplied to the steam nozzle 900 may be automatically supplied to the steam generator 810 by gravity. As a result, water supplied from the water supply pump 880 to the steam nozzle 900 has no possibility of flowing back to the water supply pump 880.

As a result, a check valve may be omitted between the water supply pump 800 and the steam nozzle 900 or the steam generator 810. Accordingly, the water supply pipe 881 may be equipped as a single water supply hose. A flow channel supplying water to the steam supply 900 may be simplified, and a possibility of water leakage from the flow channel may be reduced accordingly.

In addition, a large amount of water may be supplied to the steam nozzle 900 at a considerable pressure via the water supply pump 800. As a result, accumulated foreign substances, bacteria being propagated, or the like in the steam nozzle 900 may be washed away by water supplied to the steam nozzle 900 and washed away into the steam generator 810. Accordingly, the steam nozzle 900 may always be maintained in a clean state, and the flow of supplied steam may be prevented from being obstructed.

In one example, when steam supplied from the steam nozzle 900 is condensed, condensed water may be re-introduced into the steam generator 810 via the recovery pipe 815. That is, condensate generated from the steam nozzle 900 may be recovered to the steam generator 810 in the same direction as the movement of water supplied from the water supply pump 800, and may be recycled as water that generates the steam. Therefore, the laundry treating apparatus of the present disclosure may prevent the water level of the water supply tank 30 from rapidly decreasing, and may also prevent waste of water.

FIG. 27 is a schematic diagram showing a flow channel structure of a steam supply of the present disclosure.

Water supplied from the water supply pump 880 may be supplied to the steam nozzle 900 along the water supply pipe 881.

The steam nozzle 900 may supply the received water to the steam generator 810.

The steam generator 810 may receive water via the recovery pipe 815.

When water is heated by the heater assembly 840 in the steam generator 810 to generate steam, the steam may be supplied to the steam nozzle 900 along the steam pipe 813.

In this regard, the recovery pipe 815 and the steam pipe 813 may be separated from each other and disposed in the steam generator 810.

Steam condensed in the steam nozzle 900 may be re-introduced into the steam generator 810 via the recovery pipe 815.

The steam generator 810 may omit a hose connected to the outside except for the recovery pipe 815 and the steam pipe 813 connected to the steam nozzle 900. That is, because the water supply pump 880 may not be directly connected to the steam generator 810, a hose connecting the water supply pump 880 with the steam generator 810 may be omitted.

Therefore, because the laundry treating apparatus of the present disclosure only needs to have the water supply pipe 881, the recovery pipe 815, and the steam pipe 813 as the flow channels related to steam, the flow channel structure may be simplified.

In addition, the steam nozzle 900 may be disposed upward of the steam generator 810. Therefore, water supplied to the steam nozzle 900 may be completely guided to the steam generator 810 without a separate check valve.

Therefore, when water supplied from the water supply pump 880 is supplied to the steam nozzle 900, all of water supplied to the steam nozzle 900 is guided to the steam generator 810, so that a possibility of water flowing back from the steam nozzle 900 to the water supply pump 880 is blocked.

Therefore, the check valve may be omitted between the water supply pump 880 and the steam nozzle 900.

In addition, because the check valve does not need to be installed on the water supply pipe 880, the water supply pipe 880 may be equipped as a single hose without needing to include a plurality of water supply pipes. As a result, not only may be the flow channel structure further simplified, but also installation and repair of the flow channel may be simplified, and a possibility of water leakage may be further reduced.

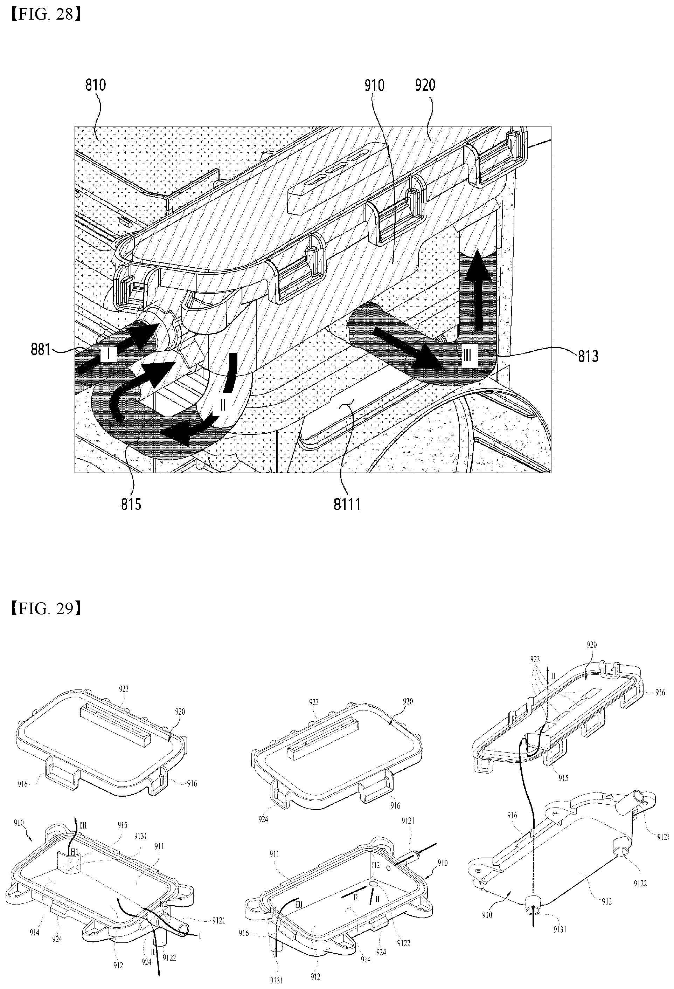

FIG. 28 shows a flow channel structure of the steam nozzle.

The steam nozzle 900 may include a supply container 910 that may receive steam from the steam casing 810 or receive water from the water supply pump 880 and accommodate steam or water therein.

The supply container 910 may be formed in a casing shape having an internal space that may receive water or steam and accommodate the same therein.

The water supply pipe 881 may be connected to the supply container 910 at a location higher than a bottom surface of the supply container 910. For example, the water supply pipe 881 may be coupled to a side surface of the supply container 910. As a result, water supplied to the supply container 910 may be blocked from flowing back into the water supply pipe 881.

The water supply pipe 881 may be connected to the supply container 910 at a location higher than the recovery pipe 815. For example, the recovery pipe 815 may have one end coupled to the bottom surface of the supply container 910 and the other end coupled to the steam generator 810. As a result, water supplied to the water supply pipe 881 may be automatically introduced into the recovery pipe 815.

In addition, the recovery pipe 815 may be formed in a U-shape so as to accommodate a certain amount of water therein.

In one example, the recovery pipe 815 may be disposed adjacent to the water supply pipe 881. As a result, water supplied to the water supply pipe 811 may be quickly introduced into the steam casing 810 without remaining in the supply container 810.

In one example, the steam pipe 813 may have one end coupled to a lower portion of the supply container 910 and the other end coupled to an upper end of the steam generator 810 or the steam cover 820. Accordingly, steam generated from the steam generator 810 may be automatically supplied to the supply container 910 by a density difference.

The steam pipe 813 may be connected to a location of the supply container 910 higher than the recovery pipe 815. In addition, the steam pipe 813 may be disposed further away from the water supply pipe 881 than the recovery pipe 815.

Accordingly, water supplied to the supply container 910 may be supplied more to the recovery pipe 815 without flowing back to the steam pipe 813.

The steam pipe 813 may be coupled to the bottom surface of the supply container 910.

The bottom surface of the supply container 910 may be constructed such that a portion where the recovery pipe 815 is coupled is lower and a portion where the steam pipe 813 is coupled is higher. To this end, a step may be formed on the bottom surface of the supply container 910 or the bottom surface may be disposed at an angle.

The steam nozzle 900 may further include a container cover 920 coupled to the upper portion of the supply container 910. The container cover 920 may include a steam spray hole 923 defined through an upper portion thereof. The steam spray hole 923 may be in communication with the interior of the inner casing 200.

The supply container 910 may be formed in a box shape with an open upper portion, and the container cover 920 may shield the upper portion of the supply container 910.

Steam supplied via the steam pipe 813 may be discharged via the steam spray hole 923 and supplied into the inner casing 200.

The steam pipe 813 may be disposed between the steam spray hole 923 and an inner surface of the supply container 910. The steam spray hole 923 may be defined between the water supply pipe 881 and the steam pipe 813, and may be defined between the recovery pipe 815 and the steam pipe 813. Accordingly, condensed steam that has not been discharged via the steam spray hole 923 may not remain inside the supply container 910 and may be discharged via the recovery pipe 815.

The steam spray hole 923 may be positioned at a center in a width direction of the container cover 920.

The container cover 920 may be coupled to the supply container 910 via hook coupling or the like. As a result, a separate fastening member that couples the container cover 920 with the supply container 910 may be omitted.

In one example, because the supply container 910 has the open top, the supply container 910 may be advantageous to install various shapes or structures inside, and the container cover 920 may be advantageous to install various structures at a bottom.

Based on the steam spray hole 923, the water supply pipe 881 and the recovery pipe 815 are positioned at one side of the supply container 910, and the steam pipe 813 is positioned at the other side of the supply container 910.

That is, the water supply pipe 881 and the recovery pipe 815 may be disposed adjacent to each other, and the steam pipe 813 may be disposed further away from the water supply pipe 881 than the recovery pipe 815.

Therefore, water flowing to the water supply pipe 881 may be supplied to the supply container 910 along a direction I. Water supplied to the supply container 910 may be immediately discharged to the recovery pipe 815 along a direction II and supplied to the steam generator 810. Steam supplied from the steam pipe 813 may be supplied to the supply container 910 along a direction III and discharged via the steam spray hole 932, and condensed water may be discharged to the recovery pipe 815 along the direction II and re-supplied to the steam generator 810.

FIG. 29 shows an embodiment of the steam nozzle.

Referring to (a) in FIG. 29, the supply container 910 may include a container side surface 911 that may be coupled with the container cover 920.

A first pipe 9121 that is coupled with the water supply pipe 881 and supplied with water may extend from one side of the container side 911.

The first pipe 9121 may be formed in a shape of a pipe, be fitted with the water supply pipe 881, and be formed integrally with the container side surface 911.

The supply container 910 may include a bottom surface 912 that extends from a lower portion of the container side surface 911. The container side surface 911 and the bottom surface 912 may define an internal space that may accommodate water or steam therein.

The supply container 910 may include a second pipe 9122 that extends from the bottom surface 912, is coupled to the recovery pipe 815, and discharges water.

The second pipe 9122 may be formed in a pipe shape, be fitted with the recovery pipe 815, and be formed integrally with the bottom surface 912.

The second pipe 9122 may be formed adjacent to the container side surface 911 on which the first pipe 9121 is disposed. As a result, water supplied from the first pipe 9121 may be quickly discharged to the second pipe 9122.

In one example, the second pipe 9122 may extend from the container side surface 911. However, even in this case, the second pipe 9122 may be disposed downward of the first pipe 9121.

Referring to (a) and (b) in FIG. 29, the supply container 910 may include a third pipe 9131 that extends from the bottom surface 912 and is coupled to the steam pipe 813. The third pipe 9131 may supply steam into the supply container 910.

The third pipe 9131 may extend from the bottom surface 912 in a pipe shape to be fitted with the steam pipe 813. The third pipe 9131 may be formed integrally with the supply container 910.

The third pipe 9131 may be disposed to be spaced apart from the first pipe 9121 and the second pipe 9122. For example, the third pipe 9131 may be disposed adjacent to an opposite surface of the container side surface 911 that faces one surface of the container side surface 911 to which the first pipe 9121 is coupled.

The supply container 910 may be longer in the width direction than in the front and rear direction.

The first pipe 9121 may extend from one side surface of the supply container 910, and the third pipe 9131 may be disposed on an opposite side surface or closer to the opposite side surface than to said one side surface. As a result, the first pipe 9121 and the third pipe 9131 may be spaced apart from each other by the greatest distance.

The second pipe 9122 may be disposed closer to the first pipe 9121 than to the third pipe 9131.

The supply container 910 may further include a separation partition wall 915 that may partition the third pipe 9131 from the inside of the supply container 910.

The container cover 920 may also be longer in the width direction than in the front and rear direction. The steam spray hole 923 may be defined as a through-hole defined in the width direction in the container cover 920.

To prevent water from being discharged via the steam spray hole 923, the first pipe 9121 and the second pipe 9122 may be disposed between the container side surface 911 and the steam spray hole 923 such that water discharged from the first pipe 9121 may be discharged immediately before reaching the steam spray hole 932.

In one example, the third pipe 9131 may be disposed between the steam spray hole 923 and the container side surface 911 facing the first pipe 9121 such that discharged steam may be discharged to the steam spray hole 923 without reaching the first pipe 9121 and the second pipe 9122.

As a result, the steam spray hole 923 may be defined between the first pipe 9121 and the third pipe 9131, and the steam spray hole 923 may be defined between the second pipe 9122 and the third pipe 9131.

The separation partition wall 915 may block an inlet of the third pipe 9131 into which steam is introduced from being exposed to the bottom surface 912. The separation partition wall 915 may protrude from the inner surface of the supply container 910 so as to surround an outer circumferential surface of the third pipe 9131.

For example, the separation partition wall 915 may protrude from the bottom surface 912 and extend so as to be in contact with an inner surface of the container side surface 911.

The separation partition wall 915 may also be formed in a pipe shape that does not come into contact with the container side surface 911 but protrudes inward.

The separation partition wall 915 may block water supplied from the first pipe 9121 from being directly introduced into the third pipe 9131. As a result, even when a large amount of water is supplied from the first pipe 9121 and is not immediately discharged to the second pipe 9122, the separation partition wall 915 may prevent the water from being introduced into the third pipe 9131.

Accordingly, even when the third pipe 9131 is positioned downward of the first pipe 9121 or on the bottom surface 912, water may be prevented from flowing back into the third pipe 9131.

In addition, steam supplied from the third pipe 9131 may be guided by the separation partition wall 915 to the steam spray hole 923. Accordingly, steam discharged from the third pipe 9131 may not remain in the supply container 910 or not be in contact with accommodated water, and thus, may be blocked from unnecessary condensation.

In one example, the bottom surface of the supply container 910 may be disposed such that the second pipe 9122 is positioned lower than the third pipe 9131. A portion of the bottom surface 912 on which the third pipe 9131 is installed may be positioned higher than a portion of the bottom surface 912 on which the second pipe 9122 is installed.

A height H1 of the container side surface 911 adjacent to the third pipe 9131 may be smaller than a height H2 of the container side surface 911 adjacent to the second pipe 9122.

The bottom surface 912 may have an inclination to be lowered along the width direction of the supply container 900.

As a result, water remaining or accommodated in the supply container 910 may be automatically discharged to the second pipe 9122 by gravity.

Referring to (c) in FIG. 29, the container cover 920 may include a moisture separating plate 922 disposed on one side of the steam spray hole 923 to prevent movement of steam or water.

The moisture separating plate 922 may be formed in a plate shape protruding from the lower portion of the container cover 920.

The moisture separating plate 912 may be disposed in the front and rear direction of the container cover 920.

The moisture separating plate 922 may pre-condense a portion of steam supplied from the third pipe 9131 that is likely to condense. Water condensed on the moisture separating plate 922 may be condensed on the bottom surface 912 of the supply container 920 and discharged via the second pipe 9122.

Steam that does not pass through the moisture separating plate 922 is likely to be condensed when passing through the steam spray hole 923, or to be condensed without reaching the laundry when supplied into the inner casing 200. Because such steam generates unnecessary residual water inside the inner casing 200 or the machine room 300, it is desirable to remove such steam from inside of the steam nozzle 900.

To this end, the moisture separating plate 912 may be disposed to be in contact with steam supplied from the third pipe 9131, and may obstruct the portion of steam that is likely to condense from flowing to the steam spray hole 922.

The moisture separating plate 922 may be disposed between the steam spray hole 923 and the third pipe 9131. In addition, a width of the moisture separating plate 922 may be greater than a width or a depth in the front and rear direction of the steam spray hole 923.

Accordingly, steam discharged from the third pipe 9131 may first come into contact with the moisture separating plate 922 and then be discharged via the steam spray hole 932.

The moisture separating plate 922 may extend from the container cover 920 with a length to be spaced apart from the bottom surface 912 of the supply container 910.

The moisture separating plate 922 may also serve as a blocking plate that prevents water supplied from the first pipe 9121 from flowing to the third pipe 9131.

The supply container 910 may include a plurality of container coupling portions 916 that may be coupled with the container cover 920.

The container cover 920 may include a fastening hook 924 that is detachably coupled with the container coupling portion 916.

The container coupling portion 916 may protrude outward from the supply container 910, and the fastening hook 924 may be fixed by being caught on the container coupling portion 916.

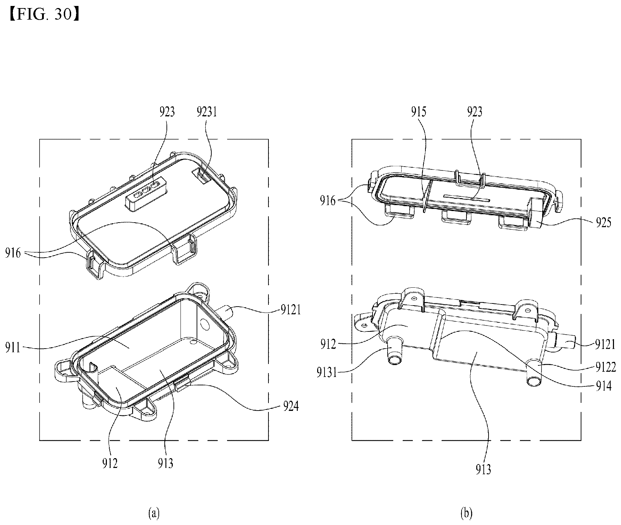

FIG. 30 shows another embodiment of the steam nozzle.

The steam nozzle 900 may include the supply container 910 having an internal space that accommodates water and steam therein, and the container cover 920 coupled to the supply container 910 to shield the internal space.

Arrangement of the first pipe 9121, the second pipe 9122, and the third pipe 9131 may be the same as that in the above-described embodiment.

Referring to (a) in FIG. 29, the bottom surface of the supply container 910 may include a first bottom surface 912 from which the third pipe 9131 extends, and a second bottom surface 913 that is formed to be lower than the first bottom surface 912 and from which the second pipe 9122 extends.

The second bottom surface 913 may be formed to extend downwardly from the first bottom surface 912 in a stepwise manner. Accordingly, water introduced from the first pipe 9131 may be restricted from flowing to the third pipe 9131 by hitting a step surface 914 formed between the first bottom surface 912 and the second bottom surface 913.

An area size of the second bottom surface 913 may be greater than an area size of the first bottom surface 912. Accordingly, even when a large amount of water is introduced from the first pipe 9131, most of the water may be accommodated only between the second bottom surface 913 and the container side surface 911 and discharged via the second pipe 9122.

In addition, the first bottom surface 912 may be formed to be inclined downward from the third pipe 9131 toward the second pipe 9122, and the second bottom surface 913 may be formed to be inclined downward toward the third pipe 9131. Accordingly, residual water existing inside the supply container 910 may be automatically discharged via the second pipe 9122.

Referring to (b) in FIG. 29, the steam spray hole 924 may be defined in a top surface of the container cover 920 to be closer to a rear surface than to a front surface of the container cover 920. The third pipe 9131 may be disposed on the bottom surface 912 to be biased rearward.

The moisture separating plate 922 may be disposed on the container cover 920 in the front and rear direction, and may be disposed between the third pipe 9131 and the steam spray hole 924.

In this regard, the moisture separating plate 922 may extend such that a protruding length thereof becomes smaller forward. For example, a cross-section thereof may be formed in a triangular shape.

Accordingly, the moisture separating plate 922 may block steam supplied from the third pipe 9131 from flowing directly to the steam spray hole 924, but may prevent the flow of steam from being unnecessarily restricted.

Therefore, steam supplied from the third pipe 9131 may be easily discharged via the steam spray hole 924 over a lower portion of the moisture separating plate 922.

In one example, water condensed on the moisture separating plate 922 may flow along a bottom cross-section of the moisture separating plate 922 and be guided to the container side surface 911.

In one example, the container cover 920 may include a discharge portion 9231 through which air or water may be discharged between the steam spray hole 924 and the first pipe 9121. Accordingly, when a high pressure is achieved inside the steam nozzle 920, some air may be discharged.

In addition, the container cover 920 may further include a lower protrusion 923 that extends downward to face the first pipe 9211.

The discharge portion 9231 may serve as a hole into which a mold forming the lower protrusion 923 is inserted.

The lower protrusion 923 may support a pipe that may be inserted or installed inside the steam nozzle 900, or may support water level and temperature sensors.

The present disclosure may be modified and implemented in various forms, so that the scope of rights thereof is not limited to the above-described embodiments. Therefore, when the modified embodiment includes elements of the claims of the present disclosure, it should be considered to fall within the scope of the present disclosure.

## Claims

1. A laundry treating apparatus comprising:
a cabinet having an opening defined at a front side thereof;
an inner casing disposed inside the cabinet to accommodate laundry therein;
a door pivotably coupled to the cabinet and configured to open and close the opening; and
a machine room including a heat supply configured to supply hot air to the inner casing and a steam supply configured to supply steam,
wherein the steam supply includes:
a water supply tank detachably disposed in the machine room to store water therein;
a steam generator accommodating therein a heater assembly configured to heat water and generate steam; and
a steam nozzle in communication with the steam generator to supply steam generated by the steam generator into the inner casing,
wherein the steam nozzle receives water accommodated in the water supply tank and delivers water to the steam generator.

2. The laundry treating apparatus of claim 1, wherein the steam nozzle is disposed upward of the steam generator.

3. The laundry treating apparatus of claim 1, wherein the steam supply further includes a recovery hose supplying water accommodated in the steam nozzle to the steam generator.

4. The laundry treating apparatus of claim 3, wherein the recovery hose has both ends disposed higher than other portions thereof.

5. The laundry treating apparatus of claim 1, wherein the steam supply further includes:
a water pump configured to supply water stored in the water supply tank to the steam nozzle; and
a water supply pipe allowing the water pump and the steam nozzle to be in communication with each other.

6. The laundry treating apparatus of claim 5, wherein the steam supply further includes a steam hose transferring steam generated from the steam generator to the steam nozzle.

7. The laundry treating apparatus of claim 1, wherein the steam nozzle includes:
a supply container providing an internal space for receiving water or steam;
a first pipe extending from the supply container to receive water; and
a second pipe extending from the supply container to deliver water to the steam generator,
wherein the second pipe is positioned downward of the first pipe.

8. The laundry treating apparatus of claim 7, wherein the steam nozzle further includes a third pipe extending from the supply container to receive steam,
wherein the third pipe is disposed downward of the first pipe.

9. The laundry treating apparatus of claim 8, wherein the supply container includes:
a container side surface having the first pipe disposed thereon and defining the internal space; and
a bottom surface extending from a lower portion of the container side surface and having the second pipe and the third pipe disposed thereon,
wherein the second pipe is disposed closer to the first pipe than to the third pipe.

10. The laundry treating apparatus of claim 8, wherein the supply container includes:
a container side surface having the first pipe disposed thereon and defining the internal space; and
a bottom surface extending from a lower portion of the container side surface and having the second pipe and the third pipe disposed thereon,
wherein the bottom surface is disposed in an inclined or stepped manner such that the second pipe is disposed lower than the third pipe.

11. The laundry treating apparatus of claim 8, wherein the supply container includes:
a container side surface having the first pipe disposed thereon and defining the internal space;
a bottom surface extending from a lower portion of the container side surface and having the second pipe and the third pipe disposed thereon; and
a separation partition wall protruding from the bottom surface to partition the second pipe and the third pipe from each other.

12. The laundry treating apparatus of claim 11, wherein the separation partition wall extends from the bottom surface to surround the third pipe.

13. The laundry treating apparatus of claim 8, wherein the steam nozzle further includes a container cover coupled to an upper portion of the supply container and transmitting steam into the inner casing,
wherein the container cover includes:
a steam spray hole discharging steam to the outside of the supply container; and
a separating plate extending from one side of the steam spray hole and spaced apart from an inner surface of the supply container to obstruct movement of steam.

14. The laundry treating apparatus of claim 13, wherein the separating plate is disposed between the second pipe and the third pipe.

15. The laundry treating apparatus of claim 14, wherein the separating plate is disposed between the steam spray hole and the third pipe.

16. The laundry treating apparatus of claim 14, wherein the separating plate is disposed closer to the third pipe than to the second pipe.
